# FASCICULE DE BREVET EUROPEEN

(11) **EP 1 689 425 B1**
(45) Date de publication et mention de la délivrance du brevet: **09.12.2009**
(21) Numéro de dépôt: 04805846.5
(22) Date de dépôt: 19.11.2004
(51) Int. Cl.: A61K 38/21, A61K 9/14, A61K 38/20, A61K 47/42

(54) **FORMULATIONS PHARMACEUTIQUES POUR LA LIBÉRATION PROLONGÉE DE PRINCIPE(S) ACTIF(S), AINSI QUE LEURS APPLICATIONS NOTAMMENT THERAPEUTIQUES**
PHARMAZEUTISCHE FORMULIERUNGEN FÜR DIE VERZÖGERTE FREISETZUNG VON EIN ODER MEHR WIRKSTOFFEN UND IHRE THERAPEUTISCHE ANWENDUNGEN
PHARMACEUTICAL FORMULATIONS FOR THE SUSTAINED RELEASE OF ONE OR MORE ACTIVE PRINCIPLES AND THERAPEUTIC APPLICATIONS THEREOF

(30) Priorité: 21.11.2003 FR 0350887
(43) Date de publication de la demande: 16.08.2006
(73) Titulaire: FLAMEL TECHNOLOGIE, Société Anonyme, 69200 Venissieux (FR)
(72) Inventeur: POULIQUEN, Gauthier, F-69007 LYON (FR); SOULA, Olivier, F-69330 MEYZIEU (FR); MEYRUEIX, Rémi, F-69009 LYON (FR); NICOLAS, Florence, F-69800 SAINT-PRIEST (FR)
(74) Mandataire: Fleurance, Raphaël
(86) Numéro de dépôt international: PCT/FR2004/050603
(87) Numéro de publication internationale: WO 2005/051416

(56) Documents cités:
- WO-A-99/18142
- WO-A-03/104303
- WO-A-2004/013206
- WO-A1-2004/060968
- WO-A1-2004/108796
- FR-A- 2 732 218
- FR-A- 2 786 098
- FR-A- 2 801 226
- FR-A- 2 822 834
- FR-A- 2 838 964

## Description

La présente invention concerne de nouvelles formulations pharmaceutiques à base de suspensions colloïdales aqueuses stables et fluides pour la libération prolongée de principe(s) actif(s), en particulier protéinique(s) et peptidique(s), ainsi que les applications, notamment thérapeutiques, de ces formulations.

Ces formulations pharmaceutiques actives concernent aussi bien la thérapeutique humaine que vétérinaire.

Dans le domaine de la libération prolongée des principes actifs pharmaceutiques notamment de protéines thérapeutiques, il existe un besoin, dans beaucoup de cas, de reproduire au mieux chez le patient une concentration plasmatique en protéine ou en peptide proche de la valeur observée chez le sujet sain.

Cet objectif se heurte à la faible durée de vie des protéines dans le plasma qui conduit à injecter de façon répétée la protéine thérapeutique. La concentration plasmatique en protéine thérapeutique présente alors un profil « en dents de scie » caractérisé par des pics élevés de concentration et des minima de concentration très bas. Les pics de concentration, très supérieurs à la concentration basale chez le sujet sain, ont des effets nocifs très marqués du fait de la toxicité élevée des protéines thérapeutiques telles que l'interleukine IL2. Par ailleurs, les minima de concentration sont inférieurs à la concentration nécessaire pour avoir un effet thérapeutique, ce qui entraîne une mauvaise couverture thérapeutique du patient et des effets secondaires graves à long terme.

Aussi, pour reproduire chez le patient une concentration plasmatique en protéine thérapeutique proche de la valeur idéale pour le traitement du patient, il importe que la formulation pharmaceutique considérée permette de libérer la protéine thérapeutique sur une durée prolongée, de façon à limiter les variations de concentration plasmatique au cours du temps.

Par ailleurs, cette formulation active doit de préférence satisfaire au cahier des charges suivant, déjà connu de l'homme de l'art :
1 - libération prolongée d'une protéine thérapeutique active et non dénaturée, par exemple humaine ou synthétique, de sorte que la concentration plasmatique est maintenue au niveau thérapeutique ;
2 - forme liquide suffisamment fluide pour être aisément injectable et stérilisable par filtration sur des filtres dont la taille des pores est inférieure ou égale à 0,2 microns ;
3 - forme liquide stable ;
4 - biocompatibilité et biodégradabilité ;
5 - non toxicité ;
6 - non immunogénicité ;
7 - excellente tolérance locale.

Pour tenter d'atteindre ces objectifs, plusieurs approches ont déjà été proposées dans l'art antérieur.

Dans la première approche, la protéine thérapeutique native est modifiée par greffage covalent d'une ou de plusieurs chaînes de polymère ou encore par greffage covalent d'une protéine telle que l'albumine sérique humaine (HSA). La protéine ainsi modifiée a une moindre affinité pour ses récepteurs et son temps de demi vie dans la circulation générale augmente considérablement. L'amplitude de la variation de concentration entre les pics et les creux de concentration plasmatique en protéine est ainsi considérablement réduite. Ainsi la société Shering Plough commercialise sous le nom VIRAFÉRON® PEG un interféron alpha 2b modifié chimiquement par greffage d'une chaîne de polyéthylène glycol de masse 12kD. Cette modification chimique se traduit par une augmentation du temps de demi vie chez le patient de 6,8 à 33 heures. Cette démarche de modification chimique de la protéine thérapeutique présente, de façon générale, deux inconvénients majeurs. Tout d'abord, la modification irréversible de la protéine, qui, n'étant plus une protéine humaine, peut conduire à long terme à des problèmes de toxicité et d'immunogénicité. Le deuxième inconvénient provient de la perte partielle de bioactivité de la protéine thérapeutique modifiée.

Dans une deuxième approche, il a été proposé d'augmenter la durée d'action grâce à des formulations comportant au moins un polymère et un principe actif, liquides à température et atmosphère ambiantes, injectables et devenant plus visqueuses après injection, par exemple sous l'effet d'un changement de pH et/ou de température.

Ainsi dans ce registre, le brevet US-B-6 143 314 divulgue une solution organique polymère à libération contrôlée de PA, formant un implant solide après injection. Cette solution comprend:
o (A) 10 à 80 % en poids d'un polymère thermoplastique de base, biocompatible, biodégradable et insoluble dans l'eau ou les fluides physiologiques (par exemple PolyLactique et/ou PolyGlycolique) ;
o (B) un solvant organique, tel que la N-MéthylPyrrolidone se dispersant dans les fluides physiologiques ;
o (C) un principe actif (PA) ;
o (D) et enfin 1 à 50 % en poids d'un agent de libération contrôlée constitué par un copolymère bloc de type PolyLactiqueGlyeolique / PolyBthylèneGlycol.
Après injection, (B) se disperse ou se dissipe dans les fluides physiologiques. (A) forme un implant encapsulant (C) qui n'est pas lié de façon covalente ni à (A) ni à (D) et qui se libère alors lentement in vivo.
Le principal inconvénient de cette technique est d'utiliser un solvant organique (B), potentiellement dénaturant pour le PA (C) (e.g. protéines thérapeutiques) et toxique pour le patient. En outre, l'hydrolyse in vivo du polymère (A) génère un acide qui peut conduire à des problèmes de tolérance locale.

Les demandes PCT WO-A-99/18142 & WO-A-00118821 concernent des solutions aqueuses de polymères qui contiennent un PA sous forme dissoute ou colloïdale, qui sont administrables à des animaux à sang chaud, notamment par injection et qui forment un dépôt de PA (e.g insuline) gélifié in vivo, car la température physiologique est supérieure à leur température de gélification. Le gel ainsi formé libère le PA de façon prolongée. Ces polymères biodégradables particuliers sont des triblocs ABA ou BAB avec A = polylactique-coglycolique (PLAGA) ou polylactique (PLA) et B = PolyEthylèneGlycol. Les températures de transformation liquide ⇒ gel de ces polymères triblocs sont par exemple de 36, 34, 30 et 26 °C. A l'instar des polymères (A) selon l'US-B-6 143 314, l'hydrolyse de ces polymères triblocs ABA ou BAB in vivo conduit à des acides qui peuvent ne pas être correctement tolérées localement.

La demande PCT WO-A-98/11874 décrit des formulations pharmaceutiques comprenant un principe actif lipophile, un polymère gélifiant (Gelrite® = gomme gellane désacétylée ou éthylhydroxycellulose) et un surfactant. L'interaction polymère/surfactant et éventuellement, s'agissant du polymère Gelrite®, la seule présence d'électrolytes tels que des ions Ca⁺⁺ en concentration physiologique conduit à la formation d'un gel constitué par un agrégat polymére/surfactant, auquel se lie de façon non covalente le principe actif lipophile. Cette formulation est destinée à une administration locale dans un organe cible (oeil e.g.). L'association agrégat/principe actif qui se forme in situ, permet la libération lente du principe actif dans l'organe cible.

Une troisième approche mise en oeuvre pour tenter de prolonger la durée d'action d'une protéine tout en conservant sa bioactivité, fut d'utiliser une protéine thérapeutique non dénaturée et de l'incorporer dans des microsphères ou des implants à base de polymères biocompatibles. Cette approche est notamment illustrée par le brevet US-B-6 500 448 et la demande US-A-2003/0133980 qui décrivent une composition à libération prolongée d'hormone de croissance humaine (hGH) dans laquelle, la protéine hormonale, préalablement stabilisée par complexation avec un métal, est ensuite dispersée dans une matrice polymère biocompatible. Le polymère biocompatible est par exemple un poly(lactide), un poly(glycolide) ou un copolymère poly(lactide-co-glycolide). La composition se présente par exemple sous la forme d'une suspension de microsphères dans une solution de carboxyméthylcellulose de sodium. Cette approche présente plusieurs inconvénients : tout d'abord, au cours du procédé de fabrication des microsphères, la protéine est mise au contact de solvants organiques potentiellement dénaturants. En outre, les microsphères sont d'une taille élevée (1 à 1000 microns), ce qui constitue une contrainte en termes d'injection et de stérilisation aisée sur filtres. Enfin, des problèmes de tolérance locale peuvent survenir lors de l'hydrolyse in situ du polymère.

Selon une quatrième approche, ont été développées des formes à libération prolongée de protéine thérapeutique constituées par des suspensions, liquides et peu visqueuses due nanoparticules chargées en protéines thérapeutiques. Ces suspensions ont permis l'administration aisée de protéines thérapeutiques natives.

Le brevet FR-B-2 822 834 décrit une suspension colloïdale de particules submicroniques susceptibles d'être utilisées, notamment pour la vectorisation de principe(s) actif(s) (PA), ces particules étant des arrangements supramoléculaires individualisés, ces particules étant à base d'au moins un copolymère amphiphile comprenant au moins un bloc de polymère hydrophile du type polyalkylèneglycol (PAG), de préférence polyéthylène-glycol (PEG) et au moins un copolyaminoacide (PAA) amphiphile linéaire, à enchaînements α-peptidiques. Ce brevet divulgue spécifiquement une suspension colloïdale obtenue en mélangeant 10 mg de copolymère amphiphile à 1 ml d'une solution d'insuline à 1,4 mg/ml à pH 7,4.

Une première forme de suspensions nanoparticulaires de libération prolongée est celle constituée par des suspensions de liposomes dans lesquelles la protéine thérapeutique native non modifiée est encapsulée. Après injection, la protéine est libérée progressivement des liposomes, ce qui prolonge le temps de présence de la protéine dans la circulation générale. Ainsi par exemple, Frossen et al, décrivent dans l'article Cancer Res 43 p 546, 1983 l'encapsulation d'agents anti-néoplastiques dans des liposomes afin d'en accroître l'efficacité thérapeutique. La libération des agents est cependant trop rapide pour obtenir une réelle libération prolongée. La société Liposome Company Inc, dans son brevet US-B-5 399 331, propose d'améliorer le temps de libération in vitro de l'interleukine 2 en la greffant de façon covalente au liposome. On retombe alors dans les travers de l'approche "protéine modifiée" évoqués ci-dessus.

Afin de pallier le manque de stabilité des liposomes, tout en gardant les avantages d'une formulation nanoparticulaire liquide et de basse viscosité, la société Flarnel Technologies a proposé une autre voie, dans laquelle la protéine thérapeutique est associée à des nanoparticules d'un polyaminoacide hydrosoluble "modifié hydrophobe", c'est-à-dire modifié par greffage de groupements hydrophobes. Ce polymère est choisi, en particulier, parmi les polyaminoacides (polyglutamates ou polyaspartates) porteurs de greffons hydrophobes.

Un des intérêts notables de ces polymères modifiés hydrophobes est de s'auto assembler spontanément dans l'eau pour former des nanoparticules.

Un autre intérêt de ces systèmes est que les protéines ou les peptides, en particulier les protéines thérapeutiques, s'associent spontanément avec les nanoparticules de polymères modifiés hydrophobes. Cette association est non covalente et s'effectue sans avoir recours à un tensioactif ni à un procédé de transformation potentiellement dénaturant. Il ne s'agit pas d'une encapsulation de la protéine dans une microsphère, comme divulgué dans le brevet US-B-6 500 448 et la demande US-A-2003/0133980. De façon totalement différente, ces nanoparticules de copolyaminoacides modifiés hydrophobes adsorbent spontanément les protéines en solution, sans les modifier chimiquement ni les dénaturer et sans leur faire subir des étapes de traitement agressives du type "mise en émulsion" et "évaporation de solvant". Les formulations peuvent être stockées sous forme liquide ou lyophilisée.
Après injection, par exemple par voie sous cutanée, ces suspensions de nanoparticules chargées en protéines libèrent progressivement la protéine non dénaturée et bioactive in vivo. De telles associations non covalentes principe actif (PA) protéinique / poly[Glu] ou poly[Asp] sont divulguées dans la demande de brevet WO-A-00/30618.
Cette demande décrit notamment des suspensions colloïdales de pH 7,4 comprenant des associations d'insuline humaine avec des nanoparticules de polyglutamate "modifié hydrophobe". Le tableau ci-dessous rend compte des polyaminoacides "modifiés hydrophobes" mis en oeuvre et des taux d'association obtenus dans les exemples du WO-A-00/30618.

| EXEMPLE | POLYMERE | Taux d'association (%) |
|---|---|---|
| 1 | poly[(GJu-0-Na)_{0,63}bloc(Glu-O-méthyle)_{0,37}] | 55 |
| 2 | poly[Glu-O-Na)_{0,66}-bloc-(Glu-O-éthyle)_{0,34}] | 26 |
| 3 | poly(Glu-O-Na)_{0,65}-bloc-(Glu-O-hexadécyle)₀,₃₅] | 36 |
| 4 | poly[(Glu-O-Na)_{0,88}-bloc-(Glu-O-dodécyle)_{0,12}] | > 90 |

Ces suspensions colloïdales titrent 1,4 mg/ml d'insuline et 10 mg/ml de polyaminoacide "modifié hydrophobe".
Il ressort de la figure 1 du WO-A-00/30618 que la durée de libération in vivo de l'insuline vectorisée par les suspensions susvisées, est de 12 H. Cette durée de libération gagnerait à être augmentée.
Ainsi, même si cette demande PCT représente déjà un progrès considérable, son contenu technique peut encore être optimisé au regard du cahier des charges énoncé ci-dessus et surtout au regard de l'allongement de la durée de libération in vivo.

Les demandes de brevet français non publiées N^{os} 02 07008 du 07/06/2002, 02 09670 du 30/07/2002, 03 50190 du 28/05/2003 et 01 50641 du 03/10/2003, concernent de nouveaux polyaminoacides amphiphiles, hydrosolubles et comprenant des unités acide aspartique et/ou des unités acide glutamique, dans lesquels au moins une partie de ces unités sont porteuses de greffons hydrophobes. A l'instar des polyaminoacides modifiés hydrophobes divulgués dans la demande WO-A-00/30618, ces nouvelles matières premières polymères forment spontanément en milieu liquide aqueux des suspensions colloïdales de nanoparticules qui peuvent être utilisées pour la libération prolongée de PA (insuline). Elles sont biocompatibles, biodégradables et les protéines, en particulier les protéines thérapeutiques s'adsorbent spontanément sur ces nanoparticules sans subir de modification chimique ou de dénaturation.
Ces demandes visent aussi de nouvelles compositions pharmaceutiques, cosmétiques, diététiques ou phytosanitaires à base de ces polyaminoacides.

Les polyaminoacides "modifiés hydrophobes" amphiphiles selon la demande de brevet français N° 02 07008 comprennent des unités acide aspartique et/ou des unités acide glutamique, porteuses de greffons hydrophobes comportant au moins un motif alpha-tocophérol, e.g. : (polyglutamate ou polyaspartate greffé par l'alpha tocophérol d'origine synthétique ou naturelle).
Cette demande non publiée divulgue spécifiquement une suspension colloïdale qui contient des nanoparticules formées par des associations polymère/protéine active et qui est obtenue en mélangeant 1 mg d'un polyglutamate greffé par l'alpha-tocophérol et 7 mg d'insuline dans 1 ml d'eau, à pH 7,0.

Les polyaminoacides "modifiés hydrophobes" amphiphiles selon la demande de brevet français N° 02 09670 comprennent des unités acide aspartique et/ou des unités acide glutamique, porteuses de greffons hydrophobes comportant au moins un motif hydrophobe reliés aux unités acide aspartique et/ou acide glutamiques par l'intermédiaire d'une rotule contenant deux fonctions amides, et plus précisément via un "espaceur" de type lysine ou ornithine.
Cette demande non publiée divulgue spécifiquement une suspension colloïdale qui contient des nanoparticules formées par des associations polymère/protéine active et qui est obtenues mélangeant 10 mg d'un polyglutamate greffé avec de l'acide palmitique via un "espaceur" lysine et 200 UI d'insuline (7,4 mg) dans 1 ml d'eau, à pH 7,4.

Les polyaminoacides "modifiés hydrophobes" amphiphiles selon la demande de brevet français N° 03 50190 comprennent des unités acide aspartique et/ou des unités acide glutamique, dont certaines sont porteuses d'au moins un greffon relié à une unité acide aspartique ou glutamique, par l'intermédiaire d'un "espaceur" "acide aminé" à base de Leu, et/ou ILeu, et/ou Val, et/ou Phe, un groupement hydrophobe en C6-C30 étant relié par une liaison ester à "l'espaceur".
Cette demande non publiée divulgue spécifiquement une suspension colloïdale qui contient des nanoparticules formées par des associations polymère/protéine active et qui est obtenue en mélangeant une solution aqueuse contenant 10 mg d'un polyglutamate greffé avec un greffon -Leu-OC8, -Val-OC12 ou -Val-cholestéryle et 200 UI d'insuline (7,4 mg), par millilitre d'eau, à pH 7,4.

La demande de brevet français FR-A-01 50641 divulgue des homopolyaminoacides linéaires, amphiphiles, anioniques, comprenant des unités acide aspartique ou des unités acide glutamique dont les extrémités sont porteuses de groupements hydrophobes comportant de 8 à 30 atomes de carbone .
En particulier, les homopolyaminoacides téléchéliques "modifiés hydrophobes" sont par exemple un poly[GluONa] à extrémités PheOC18/C18 ou un poly[GluONa] à extrémités PheOC18/alpha-tocophérol. Cette demande non publiée décrit également une suspension colloïdale qui contient des nanoparticules formées par des associations polymère/protéine active et qui est obtenue en mélangeant 10 mg de l'un des polymères susvisés et 200 UI d'insuline (7,4 mg) par millilitre d'eau, à pH 7,4.
La durée de libération in vivo de l'insuline "vectorisée" par les suspensions selon ces demandes non publiées, gagnerait à être augmentée.
En tout état de cause, tout cet art antérieur sur les suspensions colloïdales de nanoparticules de polyaminoacides modifiés hydrophobes, ne révèle pas de formulation permettant :
(I) d'accroître suffisamment la durée de libération de la protéine active après injection par voie parentérale, en particulier sous cutanée ;
(II) et/ou de réduire le pic de concentration plasmatique de la protéine active après injection de la formulation la contenant.

Dans ces conditions, l'un des objectifs essentiels de la présente invention est donc de proposer une formulation pharmaceutique liquide pour la libération prolongée de principe(s) actif(s), remédiant aux carences de l'art antérieur, et en particulier permettant après injection par voie parentérale (e.g. sous cutanée), d'obtenir une durée de libération in vivo prolongée pour des principes actifs -PA-(e.g. protéines, peptides thérapeutiques et petites molécules) non dénaturés, par exemple des protéines humaines ou synthétiques.

Un autre objectif essentiel de invention est de proposer une formulation pharmaceutique liquide à libération prolongée du PA in vivo, qui soit suffisamment fluide pour être aisément injectable et stérilisable par filtration sur des filtres dont la taille des pores est inférieure ou égale à 0,2 microns.

Un autre objectif essentiel de l'invention est de proposer une formulation pharmaceutique liquide à libération prolongée du PA in vivo, qui soit stable à la conservation tant sur le plan physico-chimique que biologique.

Un autre objectif essentiel de l'invention est de proposer une formulation pharmaceutique liquide à libération prolongée du PA in vivo, qui présente au moins l'une des propriétés suivantes: biocompatibilité, biodégradabilité, atoxicité, bonne tolérance locale.

Un autre objectif essentiel de l'invention est de proposer une formulation pharmaceutique pour la libération prolongée lente de PA in vivo, cette formulation étant une suspension colloïdale aqueuse de basse viscosité comprenant des particules submicroniques de polymère PO auto-associées à au moins un PA, le polymère PO étant un polymère biodégradable, hydrosoluble et porteur de groupements hydrophobes.

Un autre objectif essentiel de l'invention est de proposer une formulation pharmaceutique de libération prolongée lente de PA in vivo, cette formulation étant une suspension colloïdale aqueuse de basse viscosité comprenant des particules submicroniques de polymère PO auto-associées à au moins un PA, le polymère PO étant, par exemple, un polyaminoacide formé par des unités acide aspartique et/ou des unités acide glutamique, au moins une partie de ces unités étant porteuses de greffons comportant au moines un groupement hydrophobe (GH), PO étant en outre biodégradable, hydrosoluble, et amphiphile.

Un autre objectif essentiel de l'invention est de proposer des produits dérivés et/ou des précurseurs de la formulation visée dans les objectifs sus énoncés.

Il est en particulier du mérite de la Demanderesse d'avoir mis au point des formulations pharmaceutiques liquides aqueuses de basse viscosité à température physiologique, qui, de façon surprenante, forment un dépôt gélifié in vivo après administration parentérale aisée chez l'homme ou les mammifères à sang chaud, la formation de ce dépôt n'étant pas déclenchée par un changement de pH, ni de température lors de l'injection parentérale, ni par la présence d'électrolyte(s) (tels que des ions Ca⁺⁺) en concentration physiologique et/ou d'au moins un tensioactif, ni encore par dispersion de solvant organique dans le milieu physiologique. Le dépôt gélifié ainsi formé augmente d'une façon significative la durée de libération du PA in vivo.
D'où il s'ensuit que l'invention concerne une formulation pharmaceutique liquide pour la libération prolongée de principe(s) actif(s) -PA-, cette formulation comprenant une suspension colloïdale, aqueuse, de basse viscosité, à base de particules submicroniques de polymère (PO) biodégradable, hydrosoluble et porteur de groupements hydrophobes (GH), lesdites particules étant associées de façon non covalente avec au moins un principe actif (PA),
**caractérisée :**
◆ **en ce que** le milieu dispersif de la suspension est essentiellement constitué par de l'eau,
◆ en ce qu'elle est apte à être injectée par voie parentérale et à former ensuite in vivo un dépôt gélifié, cette formation de dépôt gélifié, :
   o étant, d'une part, au moins en partie provoquée par au moins une protéine physiologique présente in vivo,
   o et permettant, d'autre part, de prolonger et de contrôler la durée de libération du PA in vivo, au-delà de 24 h après l'administration,
◆ en ce qu'elle est liquide dans les conditions d'injection,
◆ et en ce qu'elle est également liquide à la température et/ou au pH physiologiques, et/ou en présence :
   * d'électrolyte physiologique en concentration physiologique,
   * et/ou d'au moins un tensioactif.

Avantageusement, cette gélification in vivo ne résulte pas d'un changement de pH et/ou de température, ni de la présence d'électrolytes (Ca⁺⁺ e.g.) en concentration physiologique et/ou d'au moins un tensioactif, ni d'une dispersion in vivo d'un ou plusieurs solvants organiques éventuellement contenus dans la formulation injectée. Sans vouloir être lié par la théorie, on peut penser que les protéines physiologiques présentes in vivo dans des concentrations physiologiques, permettent l'agrégation des nanoparticules de PO associées à au moins un PA. Une telle gélification s'opère, par exemple, en une ou plusieurs heures, 24 h, 48 h ou 72 h, entre autres.

Conformément à l'invention, la concentration en [PO] de la formulation est fixée à une valeur suffisamment élevée pour permettre la formation de dépôt gélifié in vivo, après injection parentérale, en présence d'au moins une protéine physiologique.

Selon un mode de définition qui n'est plus basé sur un comportement in vivo comme ci-dessus indiqué, mais sur un comportement in vitro, l'invention concerne une formulation pharmaceutique liquide pour la libération prolongée de principe(s) actif(s) - PA-, cette formulation :
o étant liquide en atmosphère ambiante,
o étant également liquide à la température et/ou au pH physiologiques et/ou en présence :
   * d'électrolyte physiologique en concentration physiologique,
   * et/ou d'au moins un tensioactif,
o et comprenant une suspension colloïdale; aqueuse, de basse viscosité, base de particules submicroniques de polymère PO biodégradable, hydrosoluble et porteur de groupements hydrophobes GH, lesdites particules étant associées de façon non covalente avec au moins un principe actif PA, de préférence en solution aqueuse, et le milieu dispersif de la suspension étant essentiellement constitué par de l'eau,
**caractérisée en ce que** sa concentration en [PO] est fixée à une valeur suffisamment élevée pour permettre la formation de dépôt gélifié in vitro, eu présence d'au moins une protéine.

De préférence, la formulation pharmaceutique liquide selon l'invention est **caractérisée en ce que** sa concentration en [PO] est telle que :
■ [PO] ≥ 0,9.C1,
■ de préférence 20.C1 ≥ [PO] ≥ C1,
■ et mieux encore 10.C1 ≥ [PO] ≥ C1
avec C1 représentant la concentration de "*gélification induite*" des particules de PO telle que mesurée dans un test Gl.

Le dépôt gélifié obtenu après injection parentérale de la formulation permet une prolongation intéressante de la durée de libération du PA (e.g. protéine thérapeutique) ainsi qu'une réduction du pic de concentration plasmatique.
La durée de libération du PA est notamment significativement augmentée par rapport à celle des formulations de l'art antérieur, en particulier celles décrites dans les demandes de brevet PCT publiée WO-A-00/30618 et les demandes de brevet français non publiées N^{os} 02 07008, 02 09670, 03 50190 et 01 50641.

La prolongation de la durée de libération de PA in vivo induite par les formulations selon l'invention est d'autant plus appréciable que les PA (e.g. protéines thérapeutiques) sont toujours pleinement bioactifs et non dénaturés.

Dans tout le présent exposé, les arrangements supramoléculaires de polymère PO associé ou non au PA, seront indifféremment désignés par "particules submicroniques" ou "nanoparticules". Cela correspond à des particules (liquides ou solides) de diamètre hydrodynamique moyen (mesuré selon un mode opératoire Md défini infra dans les exemples) e.g. compris entre 1 et 500 nm, de préférence entre 5 et 250 nm.

En outre, il est tout à fait important de noter que ces formulations sont liquides, c'est-à-dire présentent avantageusement une viscosité très faible, qui rend leur injection aisée. Elles ne gélifient qu'in vivo.
Suivant l'invention, les qualificatifs "liquide", "basse" ou "très faible viscosité" correspondent, avantageusement, à une viscosité dynamique à 20 °C inférieure ou égale à 5 Pa.s. La mesure de référence pour la viscosité peut être réalisée, par exemple, à 20 °C à l'aide d'un rhéomètre AR1000 (TA Instruments) équipé d'une géométrie cône-plan (4 cm, 2°). La viscosité v est mesurée pour un gradient de cisaillement de 10 s⁻¹.
Ainsi, la viscosité des formulations selon l'invention peut être, par exemple, comprise entre 1.10⁻³ et 5 Pa.s, de préférence entre 1.10⁻³ et 0,8 Pa.s et, plus préférentiellement encore, entre 1.10⁻³ et 0,5 Pa.s.
Cette faible viscosité rend les formulations de l'invention non seulement aisément injectables par voie parentérale, en particulier par voie intramusculaire ou sous-cutanée, entre autres, mais aussi stérilisables aisément et à moindre coût par filtration sur des filtres de stérilisation de 0,2 µm de taille de pores.
Cet état liquide ou cette faible viscosité des formulations de l'invention existe aussi bien à des températures d'injection correspondant à des températures ambiantes, par exemple comprises entre 4 et 30 °C, qu'à la température physiologique.

La formulation selon l'invention est, de préférence, une suspension colloïdale aqueuse de nanoparticules associées à un ou plusieurs PA. Cela signifie que, conformément à l'invention, le milieu dispersif de cette suspension est essentiellement formé par de l'eau. En pratique, cette eau représente, par exemple, au moins 50 % en poids par rapport à la masse totale de la formulation.

Au sens de l'invention, le terme "protéine" désigne aussi bien une protéine qu'un peptide. Cette protéine ou ce peptide pouvant être modifié ou non, par exemple, par greffage d'un ou de plusieurs groupements polyoxyéthylèniques.

Par "protéines physiologiques", on entend, au sens de l'invention, les protéines et/ou les peptides endogènes des mammifères à sang chaud présents sur le site d'injection.

Par "température physiologique", on entend au sens de l'invention, la température physiologique des mammifères à sang chaud, à savoir par exemple environ 37-42 °C.

Par "pH physiologique", on entend, au sens de l'invention, un pH par exemple compris entre 6 et 7,6.

Par "gel", on entend, au sens de l'invention, un état semi-solide dans lequel se transforme la formulation liquide selon l'invention, et ce spontanément par la seule présence de protéine(s) physiologique(s), sans intervention essentielle du pH physiologique et/ou de la température physiologique et/ou de la présence d'un électrolyte physiologique (Ca⁺⁺ e.g.) et/ou de la dispersion (ou dissipation) in vivo d'un solvant organique éventuellement présent dans la formulation injectée.

Par "électrolyte physiologique", on entend, au sens de l'invention, tout élément électrolyte (par exemple des ions Ca⁺⁺) présent chez les mammifères à sang chaud.

Par "concentration physiologique", on entend, au sens de l'invention, toute concentration physiologique rencontrée chez les mammifères à sang chaud, pour le milieu physiologique considéré.

En outre, les formulations selon l'invention sont non toxiques, bien tolérées localement et stables.

Il est également du mérite des inventeurs d'avoir mis au point un test in vitro GI permettant de sélectionner une population des formulations préférées selon l'invention et de déterminer les concentrations idoines en PO dans les formulations.

Conformément à invention, le test GI de mesure de la concentration de gélification C1, est un test de référence permettant de définir la concentration critique C1, dénommée ci après concentration de gélification induite C1, qui caractérise chaque formulation colloïdale selon l'invention.
Le test GI de détermination de la concentration C1 de gélification induite est le suivant :
Afin de déterminer la concentration C1, on prépare des formulations colloïdales de concentrations variables en polymère amphiphile selon l'invention et de concentration constante en protéine thérapeutique. A cette fin on met en solution dans de l'eau de ionisée des quantités croissantes de poudre sèche du polymère. Les solutions sont maintenues à 25 °C sous agitation magnétique durant 16 heures avant d'être mélangées avec une solution concentrée en protéine thérapeutique. Le volume et la concentration de cette solution de protéine thérapeutique sont ajustés afin d'obtenir la concentration en protéine recherchée pour la formulation [par exemple 0, 3 mg/ml d'interféron alpha 2b ou 2,5 mg/ml d'interleukine 2 (IL2)].

Les formulations colloïdale ainsi préparées sont mélangées à une solution aqueuse d'albumine de sérum bovin (BSA) concentrée à 30 mg/ml, puis centrifugées pendant 15 minutes à 3000 t/min. Les mélanges sont laissés sous agitation douce pendant 24h avant d'être récupérés pour être caractérisés.

Les mesures de viscoélasticité sont effectuées sur un rhéomètre TA Instruments AR 1000, équipé d'une géométrie cône-plan (diamètre 4cm et angle 1,59°). Une déformation de 0,01 rad, située dans le domaine de viscoélasticité linéaire, est imposée de manière sinusoïdale sur une gamme de fréquence comprise entre 0,1 et 300 rad/s. La température de l'échantillon est maintenue constante à 20 °C par le biais d'une cellule Peltier.

Les spectres en fréquence du module élastique G' et du module visqueux ou de perte, G", permettent de définir le temps de relaxation caractéristique Tr défini ici comme l'inverse de la fréquence à laquelle le module élastique G' croise le module visqueux G". On trouvera un exposé détaillé de ces questions dans l'ouvrage Ferry, Viscoelastic Properties of Polymers, J.D.Ferry, J.Wiley, NY, 1980 et dans l'article de J. REGALADO et al. Macromolecules 1999, 32, 8580.

La mesure du temps de relaxation Tr en fonction de la concentration en polymère de la formulation permet de définir la concentration C1 à laquelle ce temps Tr croise 1 seconde. Des exemples de valeurs de la concentration de gélification C1 seront donnés dans l'exemple 8 ci après.

De la même façon, on peut définir les concentrations C0,1 et C10 pour lesquelles le temps de relaxation dépasse respectivement 0,1 s et 10s. Ces concentrations se classent dans l'ordre croissant suivant : C0,1 < C1 < C10.

Suivant une variante de la formulation selon l'invention :
➢ [PO] ≥ C0,1,
➢ de préférence [PO] ≥ C1,
➢ et plus préférentiellement encore [PO] ≥ C10.

Suivant une caractéristique additionnelle avantageuse : [PO] ≤ 20.C1

Au sens de l'invention et dans tout le présent exposée les termes "association" ou "associer" employés pour qualifier les relations entre un ou plusieurs principes actifs et les polymères PO (par exemple les polyaminoacides), signifient en particulier, que le ou les principes actifs sont liés au(x) polymère(s) PO [par exemple le (ou les) polyaminoacide(s)] par une liaison non covalente, par exemple par interaction électrostatique et/ou hydrophobe et/ou liaison hydrogène et/ou gêne stérique.

Les polymères PO selon l'invention sont des polymères biodégradables, hydrosolubles et porteurs de groupements hydrophobes GH. Les groupements hydrophobes peuvent être en nombre réduit vis à vis du reste de la chaîne et peuvent se situer latéralement à la chaîne ou intercalés dans la chaîne, et être répartis de façon aléatoire (copolymère statistique) ou répartis sous forme de séquences ou de greffons (copolymères blocs ou copolymères séquencés).

Sans vouloir se limiter les polymères PO modifiés hydrophobes peuvent être choisis dans le groupe comprenant les copolyaminoacides amphiphiles, les polysaccharides - de préférence dans le sous-groupe comprenant les pullulanes et/ou les chitosans et/ou les mucopolysaccharides-, les gélatines ou leurs mélanges.

Selon un mode préféré de réalisation de l'invention, PO est choisi parmi les copolyaminoacides amphiphiles.
Au sens de l'invention et dans tout le présent exposé, le terme «*polyaminoacide*» couvre aussi bien les oligoaminoacides comprenant de 2 à 20 unités "acide aminé" que les polyaminoacides comprenant plus de 20 unités "acide aminé".

De préférence, les polyaminoacides selon la présente invention sont des oligomères ou des homopolymères comprenant des unités récurrentes acide glutamique ou aspartique ou des copolymères comprenant un mélange de ces deux types d'unités "acide aminé". Les unités considérées dans ces polymères sont des acides aminés ayant la configuration D ou L ou D/L et sont liées par leurs positions alpha ou gamma pour l'unité glutamate ou acide glutamique et alpha ou bêta pour l'unité acide aspartique ou aspartate.

Les unités "acide aminé" préférées de la chaîne polyaminoacide principale sont celles ayant la configuration L et une liaison de type alpha.

Suivant un mode encore plus préféré de réalisation de l'invention, le polymère PO est un polyaminoacide formé par des unités acide aspartique et/ou des unités acide glutamique, au moins une partie de ces unités étant porteuses de greffons comportant au moins un groupement hydrophobe GH. Ces polyaminoacides sont notamment du type de ceux décrits dans la demande de brevet PCT WO-A-00/30618.

Selon une première possibilité, le (ou les) PO de la formulation sont définis par la formule générale (**I**) suivante : 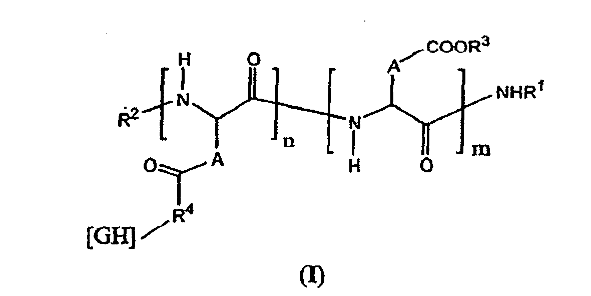 dans laquelle :
■ R¹ représente un H, un alkyle linéaire en C2 à C10 ou ramifié en C3 à C10, benzyle, une unité acide aminé terminale ou -R⁴-[GH] ;
■ R² représente un H, un groupe acyle linéaire en C2 à C10 ou ramifié en C3 à C10, un pyroglutamate ou -R⁴-[GH] ;
■ R³ est un H ou une entité cationique, de préférence sélectionnée dans le groupe comprenant :
   - les cations métalliques avantageusement choisis dans le sous-groupe comprenant : le sodium, le potassium, le calcium, le magnésium,
   - les cations organiques avantageusement choisis dans le sous-groupe comprenant :
      - les cations à base d'amine,
      - les cations à base d'oligoamine,
      - les cations à base de polyamine (la polyethylèneimine étant particulièrement préféré),
      - les cations à base d'acide(s) aminé(s) avantageusement choisis dans la classe comprenant les cations à base de lysine ou d'arginine,
   - ou les polyaminoacide cationiques avantageusement choisis dans le sous-groupe comprenant la polylysine ou l'oligolysine ;
■ R⁴ représente une liaison directe ou un "espaceur" à base de 1 à 4 unités acide aminé ;
■ A représente indépendamment un radical -CH₂- (unité aspartique) ou-CH₂-CH₂- (unité glutamique) ;
■ n/(n+m) est défini comme le taux de greffage molaire et sa valeur est suffisamment basse pour que PO mis en solution dans l'eau à pH 7 et à 25°C, forme une suspension colloïdale de particules submicroniques de PO, de préférence n/(n + m) est compris entre 1 à 25 % molaire et mieux encore entre 1 et 15 % molaire ;
■ n + m est défini comme le degré de polymérisation et varie de 10 à 1000, de préférence entre 50 et 300 ;
■ GH représente un groupement hydrophobe.

Selon une deuxième possibilité, le (ou les) PO de la formulation répond à l'une des formules générales (**II**), (**III**) et (**IV**) suivantes : 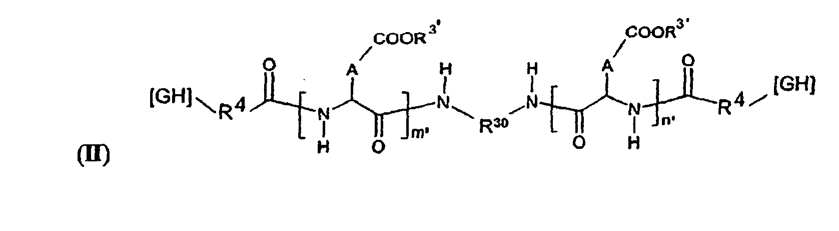 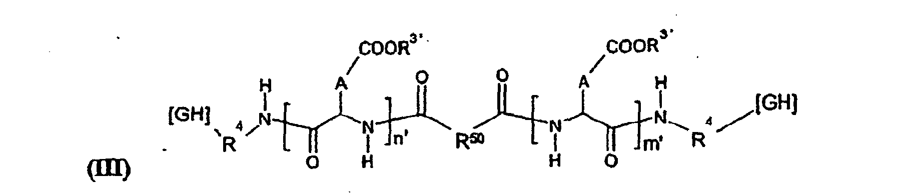 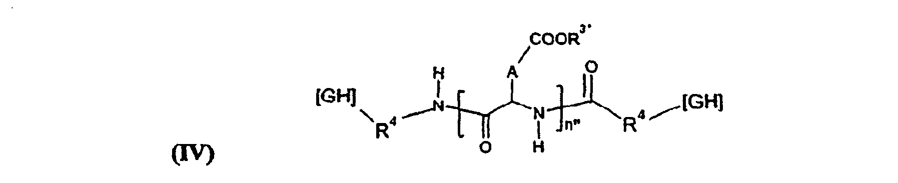 dans lesquelles :
■ GH représente un groupement hydrophobe ;
■ est un groupement alkyle linéaire en C2 à C6 ;
■ R^{3'} est un H ou une entité cationique, de préférence sélectionnée dans le groupe comprenant :
   - les cations métalliques avantageusement choisis dans le sous-groupe comprenant : le sodium, le potassium, le calcium, le magnésium,
   - les cations organiques avantageusement choisis dans le sous-groupe comprenant :
      - les cations à base d'amine,
      - les cations à base d'oligoamine,
      - les cations à base de polyamine (la polyéthylèneimine étant particulièrement préféré),
      - les cations à base d'acide(s) aminé(s) avantageusement choisis dans la classe comprenant les cations à base de lysine ou d'arginine,
   - ou les polyaminoacides cationiques avantageusement choisis dans le sous-groupe comprenant la polylysine ou l'oligolysine,
■ R⁵⁰ est un groupement alkyle, dialcoxy ou diamine en C2 à C6;
■ R⁴ représente une liaison directe ou un "espaceur" à base de 1 à 4 unités acide aminé ;
■ A représente indépendamment un radical -CH₂- (unité acide aspartique) ou -CH₂-CH₂- (unité acide glutamique);
■ n' + m' ou n"est défini comme le degré de polymérisation et varie de 10 à 1000, de préférence entre 50 et 300.

Avantageusement, les groupements GH du PO représentent chacun indépendamment les uns des autres un radical monovalent de formule suivante : 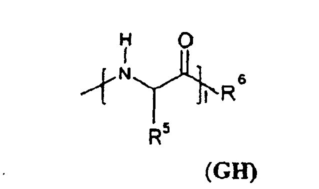 dans laquelle :
- R⁵ représente un méthyle(alanine), isopropyle (valine), isobutyle (leucine), secbutyle (isoleucine), benzyle (phénylalanine) ;
- R⁶ représente un radical hydrophobe comportant de 6 à 30 atomes de carbone;
- I varie de 0 à 6.

Selon une caractéristique remarquable de l'invention, tout ou partie des groupements hydrophobes R⁶ des PO sont choisis de façon indépendante, dans le groupe de radicaux comportant :
■ un alcoxy linéaire ou ramifié comportant de 6 à 30 atomes de carbone et pouvant comporter au moins un hétéroatome (de préférence O et/ou N et/ou S) et/ou au moins une insaturation,
■ un alcoxy comportant 6 à 30 atomes de carbone et ayant un ou plusieurs carbocycles annelés et contenant éventuellement au moins une insaturation et/ou au moins un hétéroatome (de préférence O et/ou N et/ou S),
■ un alcoxyaryle ou un aryloxyalkyle de 7 à 30 atomes de carbone et pouvant comporter au moins une insaturation et/ou au moins un hétéroatome (de préférence O et/ou N et/ou S).

En pratique et sans que cela ne soit limitatif, le radical hydrophobe R⁶ du greffon du PO est issu d'un précurseur alcoolique choisi dans le groupe comprenant: l'octanol, le dodécanol, le tétradécanol, l'hexadécanol, l'octadécanol, l'oleylalcool, le tocophérol ou le cholestérol.

Selon une première forme de réalisation de l'invention, les chaînes principales des polyaminoacides sont des homopolymères d'alpha-L-glutamate ou d'acide alpha-L-glutamique.

Selon une deuxième forme de réalisation de l'invention, les chaînes principales des polyamincacides sont des homopolymères d'alpha-L-aspartate ou d'acide alpha-L-aspartique.

Selon une troisième forme de réalisation de l'invention, les chaînes principales des polyaminoacides sont des copolymères d'alpha-L-aspartatelalpha-L-glutamate ou d'acide alpha-L-aspartique/alpha-L-glutamique.

Avantageusement, la distribution des unités acide aspartique et/ou acide glutamique de la chaîne polyaminoacide principale du PO est telle que le polymère ainsi constitué est soit aléatoire, soit de type bloc, soit de type multibloc.

Selon un autre mode de définition, le PO mis en oeuvre dans la formulation selon l'invention a une masse molaire qui se situe entre 2 000 et 100 000 g/mole, et de préférence entre 5 000 et 40 000 g/mole.

Selon une variante, le PO de la formulation selon l'invention est porteur d'au moins un greffon de type polyalkylène-glycol lié à une unité glutamate et/ou aspartate.

Avantageusement, ce greffon est de type polyalkyléne-glycol est de formule (V) suivante. 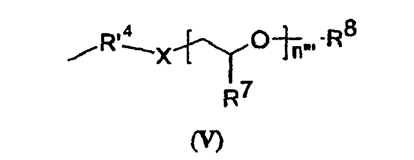 dans laquelle :
- R'⁴ représente une liaison directe ou un "espaceur" à base de 1 à 4 unités acide aminé ;
- X est un hétéroatome choisi dans le groupe comportant l'oxygène, l'azote ou un soufre ;
- R⁷ et R⁸ représentent indépendamment un H, un alkyle linéaire en C1 à C4 ;
- n"' varie de 10 à 1000, de préférence de 50 à 300.

En pratique, le polyalkylèneglycol est par exemple un polyethylène glycol.

Il est souhaitable, conformément à l'invention, que le pourcentage molaire de greffage du polyalkylène glycol varie de 1 à 30 %.

Les polyaminoacides PO sont en outre extrêmement intéressants, du fait qu'à un taux de greffage ajustable, ils se dispersent dans l'eau à pH 7,4 (par exemple avec un tampon phosphate) pour donner des suspensions colloïdales.

De plus, des principes actifs PA tels que des protéines, des peptides ou des petites molécules, peuvent s'associer spontanément à des nanoparticules comprenant ces polyaminoacides PO.

Il convient de comprendre que les PO à base de polyaminoacides contiennent des fonctions carboxyliques qui sont, soit neutres (forme COOH), soit ionisées (anion COO⁻), selon le pH et la composition. Pour cette raison, la solubilité dans une phase aqueuse est directement fonction du taux de COOH libres des PO (non greffé par le motif hydrophobe) et du pH. En solution aqueuse, le contre-cation peut être un cation métallique tel que le sodium, le calcium ou le magnésium, ou un cation organique tel que la triéthanolamine, le tris(hydroxyméthyl)-aminométhane ou une polyamine tel que la polyéthylèneimine.

Les PO de type polyaminoacides susceptibles d'être utilisés dans la formulation de l'invention sont, par exemple, obtenus par des méthodes connues de homme de l'art. Les polyaminoacides statistiques peuvent être obtenus par greffage du greffon hydrophobe, préalablement fonctionnalisé par "l'espaceur", directement sur le polymère par une réaction classique de couplage. Les PO polyaminoacides blocs ou multiblocs peuvent être obtenus par polymérisation séquentielle des anhydrides de N-carboxy-aminoacides (NCA) correspondant.

On prépare par exemple un polyaminoacide, homopolyglutamate, homopolyaspartate ou un copolymère glutamate/aspartate, bloc, multibloc ou aléatoire selon des méthodes classiques.

Pour l'obtention de polyaminoacide de type alpha, la technique la plus courante est basée sur la polymérisation d'anhydrides de N-carboxy-aminoacides (NCA), décrite, par exemple, dans l'article *"*Biopolymers, 1976, 15, 1869 et dans l'ouvrage de H.R. Kricheldorf "alpha-Aminoacid-N-carboxy Anhydrides and related Heterocycles" Springer Verlag (1987). Les dérivés d'NCA sont de préférence des dérivés NCA-O-Me, NCA-O-Et ou NCA-O-Bz (Me = Méthyl, Et = Ethyle et Bz = Benzyle). Les polymères sont ensuite hydrolysés dans des conditions appropriées pour obtenir le polymère sous sa forme acide. Ces méthodes sont inspirées de la description donnée dans le brevet FR-A-2 801 226 de la demanderesse. Un certain nombre de polymères utilisables selon l'invention, par exemple, de type acide poly(alpha-L-aspartique), acide poly(alpha-L-glutamique), acide poly(alpha-D-glutamique) et acide poly(gamma-L-glutamique) de masses variables sont disponibles commercialement. L'acide polyaspartique de type alpha-bêta est obtenu par condensation de l'acide aspartique (pour obtenir un polysuccinimide) suivie d'une hydrolyse basique (cf. Tomida et al. Polymer 1997,38,4733-36).

Le couplage du greffon avec une fonction acide du polymère est réalisé aisément par réaction du polyaminoacide en présence d'un carbodiimide comme agent de couplage et optionnellement, un catalyseur tel que la 4-diméthylaminopyridine et dans un solvant approprié tel que le diméthylformamide (DMF), la N-méthyl pyrrolidone (NMP) ou le diméthylsulfoxide (DMSO). Le carbodiimide est par exemple, le dicyclohexylcarbodiimide ou le diisopropylcarbodiimide. Le taux de greffage est contrôlé chimiquement par la stoechiométrie des constituants et réactifs ou le temps de réaction. Les greffons hydrophobes fonctionnalisés par un "espaceur" sont obtenus par couplage peptidique classique ou par condensation directe par catalyse acide. Ces techniques sont bien connues de l'homme de l'art.

Pour la synthèse de copolymère bloc ou multibloc, on utilise des dérivés NCA préalablement synthétisés avec le greffon hydrophobe. Par exemple, le dérivé NCA-hydrophobe est copolymérisé avec le NCA-O-Benzyl puis on enlève par hydrolyse sélectivement les groupements benzyliques.

La synthèse de polyaminoacides PO conduit préférablement à des suspensions aqueuses de nanoparticules de PO.

De telles suspensions peuvent être transformées en poudres de nanoparticules de PO par séchage, de manière appropriée et connue de l'homme de l'art, comme par exemple : chauffage (étuve....), mise sous vide, utilisation de dessiccants, lyophilisation, atomisation.

Ces nanoparticules de PO, en suspension ou à l'état pulvérulent, forment une matière première pour la préparation des formulations selon l'invention.

A ce propos, il peut être précisé que les formulations selon l'invention résultent de l'association non covalente de nanoparticules à base d'au moins un PO et d'au moins un PA, dans un milieu liquide aqueux.

Pour la préparation, le PO et/ou le PA peut être sous forme solide (de préférence poudre) et/ou sous forme liquide (de préférence suspension aqueuse colloïdale).

L'association PA/PO signifie au sens du présent exposé que le (ou les) PA est (sont) assticié(s) au(x) polymère(s) PO [e.g. un ou plusieurs polyaminoacide(s)] par une ou plusieurs liaisons autre(s) qu'une (ou que des) liaison(s) chimique(s) covalente(s).

Les techniques d'association d'un ou de plusieurs PA aux PO selon l'invention, sont décrites notamment dans la demande de brevet WO-A-00/30618. Elles consistent à incorporer au moins un PA dans le milieu liquide contenant des nanoparticules de PO, de manière à obtenir une suspension colloïdale de nanoparticules chargées en ou associées avec un ou plusieurs principe(s) actif(s).

L'invention a donc également pour objet un procédé de préparation de la formulation susvisée.

Selon un premier mode préféré de mise en oeuvre, ce procédé est **caractérisé en ce qu**'il consiste essentiellement :
◆ à mettre en oeuvre une suspension colloïdale de nanoparticules d'au moins un PO,
◆ à mélanger cette suspension colloïdale de nanoparticules de PO avec au moins un PA, de préférence en solution aqueuse,
◆ à ajouter éventuellement au moins un excipient,
◆ au besoin à ajuster le pH et/ou l'osmolarité et,
◆ éventuellement à filtrer la suspension ainsi obtenue.

Avantageusement, le (ou les) PA(s) est (sont) sous forme de suspension ou de solution aqueuse pour le mélange avec la suspension colloïdale de nanoparticules de PO.

Selon un second mode de mise en oeuvre, ce procédé est **caractérisé en ce qu**'il consiste essentiellement :
◆ à mettre en oeuvre une poudre d'au moins un polymère PO,
◆ à mélanger cette poudre avec une suspension ou solution aqueuse d'au moins un PA, de préférence en solution aqueuse,
◆ à ajouter éventuellement au moins un excipient,
◆ au besoin à ajuster le pH et/ou l'osmolarité et,
◆ éventuellement à filtrer la suspension ainsi obtenue.

Les formulations ainsi obtenues peuvent également être mises en formes de gels, de poudre ou de film par les méthodes classiques connues de l'homme de l'art, telles que la concentration par diafiltration ou évaporation, le couchage, l'atomisation ou la lyophilisation, entre autres. Ces méthodes peuvent être éventuellement combinées.

D'où il s'ensuit un troisième mode de mise en oeuvre du procédé de préparation des formulations liquides selon l'invention, ce troisième mode consistant essentiellement :
◆ à mettre en oeuvre une poudre issue du séchage de la formulation liquide selon l'invention telle que définie ci-dessus,
◆ à mélanger cette poudre avec un milieu liquide aqueux, de préférence sous agitation,
◆ à ajouter éventuellement au moins un excipient,
◆ au besoin à ajuster le pH et/ou l'osmolarité et,
◆ éventuellement à filtrer la suspension ainsi obtenue.

Les excipients susceptibles d'être rajoutés sont par exemple des antimicrobiens, des tampons, des antioxydants, des agents permettant d'ajuster l'isotonicité qui sont connus de l'homme de l'art. On pourra par exemple se référer à l'ouvrage : *Injectable Drug Development,* P.K. Gupta et al., Interpharm Press, Denver, Colorado 1999.

La filtration éventuelle de la formulation liquide sur des filtres de porosité égale, par exemple, à 0,2 µm, permet de la stériliser. Elle peut être ainsi directement injectée à un patient.

Tous ces exemples de préparation de formulations liquides selon l'invention sont avantageusement réalisés en atmosphère et à température ambiantes (25°C e.g.).

Selon un autre de ses aspects, l'invention englobe tout produit dérivé obtenu à partir de la formulation liquide selon l'invention telle que définie supra et comprenant des particules submicroniques, formées par des associations non covalentes PO / PA telles que définies ci-dessus.
En pratique, ces produits dérivés peuvent notamment être constitués par des poudres, des gels, des implants ou des films, entre autres.

En outre, l'invention vise tout précurseur de la formulation liquide injectable telle que définie supra.

S'agissant toujours de ces produits dérivés, il doit être souligné que l'invention concerne également un procédé de préparation d'une poudre dérivée de la formulation telle que définie supra, ce procédé étant **caractérisé en ce que** ladite poudre est obtenue par séchage de la formulation telle que définie ci-dessus.

Selon l'invention, le PA peut être une protéine, une glycoprotéine, une protéine liée à une ou plusieurs chaînes polyalkylèneglycol [de préférence PolyÉthylèneGlycol (PEG) : "protéine-PEGylée"], un polysaccharide, un liposaccharide, un oligonucléotide, un polynucléotide ou un peptide.

De préférence, le PA est sélectionné parmi les hémoglobines, les cytochromes, les albumines, les interférons, les cytokines, les antigènes, les anticorps, l'érythropoïétine, l'insuline, les hormones de croissance, les facteurs VIII et IX, les interleukines ou leurs mélanges, les facteurs stimulants de l'hématopoïèse et leurs mélanges.

Selon une variante, le principe actif est une "petite" molécule organique hydrophobe, hydrophile ou amphiphile du type de celles appartenant à la famille des anthracyclines, des taxoïdes ou des camptothécines ou du type de celles appartenant à la famille des peptides telles que la leuprolide ou la cyclosporine et leurs mélanges
Au sens du présent exposé, une "petite" molécule est notamment une petite molécule non protéinique.

Suivant une disposition intéressante de la formulation selon l'invention qui concerne tous les PA à l'exclusion des petites molécules, la fraction massique de principe actif PA non associé aux nanoparticules de polymère PO, est telle que (exprimée en % par rapport à la masse totale de la formulation):
➢ [PA non associé] ≤ 1
➢ de préférence [PA non associé] ≤ 0,5.

Parmi les qualités primordiales de la formulation selon l'invention, figurent son caractère injectable et à sa capacité à former un dépôt sur le site d'injection, in vivo, par gélification ou encore par agrégation des nanoparticules, en présence de protéines physiologiques ou analogues.

La formulation selon l'invention peut notamment être injectée par voie parentérale, sous-cutanée, intramusculaire, intradermique, intrapéritonéale, intracérébrale ou dans une tumeur.

La formulation selon l'invention peut aussi être administrée par voie orale, nasale, vaginale, oculaire ou buccale.

Avantageusement, la formulation est destinée à la préparation de médicaments, en particulier pour administration parentérale, sous-cutanée, intramusculaire, intradermique, intrapéritonéale, intracérébrale ou dans une tumeur, voire par voie orale, nasale, vaginale ou oculaire.

Bien que la formulation selon l'invention soit de préférence pharmaceutique, cela n'exclut pas pour autant les formulations cosmétiques, diététiques ou phytosanitaires comprenant au moins un PO tel que défini ci-dessus et au moins un principe actif correspondant.

Selon encore un autre de ses aspects, l'invention vise un procédé de préparation de médicaments, en particulier pour administration parentérale, sous-cutanée, intramusculaire, intradermique, intrapéritonéale, intracérébrale ou dans une tumeur, voire par voie orale, nasale, vaginale ou oculaire, **caractérisé en ce qu**'il consiste essentiellement à mettre en oeuvre au moins une formulation sus-définie et/ou tout produit dérivé et/ou tout précurseur de ladite formulation.

L'invention concerne également l'utilisation thérapeutique de la formulation telle que décrite dans le présent exposé, consistant essentiellement à administrer la-dite formulation par voie parentérale, sous-cutanée, intramusculaire, intradermique, intrapéritonéale, intracérébrale ou dans une tumeur, voire par voie orale, nasale, vaginale ou oculaire, de manière à ce qu'elle forme un dépôt gélifié/réticulé sur le site d'injection.

L'invention sera mieux comprise et ses avantages et variantes de mise en oeuvre ressortiront bien des exemples qui suivent et qui décrivent la synthèse des PO formés par des polyaminoacides greffés par un groupement hydrophobe, leur transformation en système de libération prolongée de PA, à savoir en formulation selon l'invention (suspension colloïdale aqueuse stable) et la démonstration de la capacité d'un tel système non seulement de s'associer à une protéine thérapeutique, mais surtout à gélifer/réticuler pour libérer de manière très prolongée in vivo la protéine thérapeutique.

### DESCRIPTION DES FIGURES

**Figure 1** : Courbes des Concentrations plasmatiques d'IFN (picogramme/ml) relevées chez le chien après injection sous cutanée
   - de la formulation d'IFN (A) selon l'invention (exemples 9 & 10):
      → courbe -■-■-
   - et de la formulation d'IFN (D) témoin hors de l'invention (exemple 10) :
      → courbe -▲-▲-,
   en fonction du temps T en heures et à une dose d'IFN de 60 µg/kg.

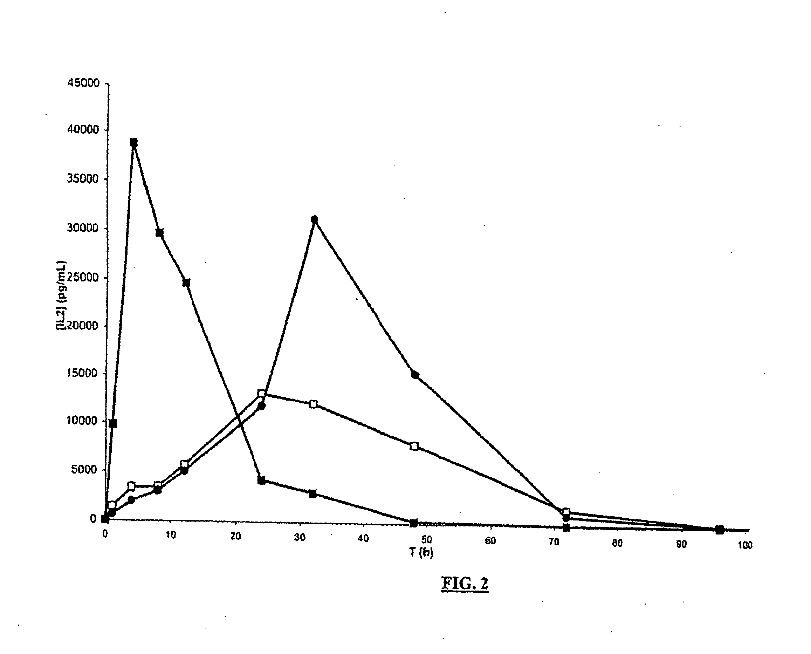

**Figure 2** : Courbes des Concentrations plasmatiques d'IL2 (picogramme/ml) relevées chez le singe après injection sous cutanée
   - de la formulation d'IL2 selon l'invention (exemple 11):
      → courbe -□-□-
   - de la formulation d'IL2 (F) témoin hors de l'invention (exemple 11):
      → courbe -●-●-,
   - et de la formulation d'IL2 (G) témoin hors de l'invention (exemple 11):
      → courbe -■-■-,
   en fonction du temps T en heures et à une dose d'IL2 de 0,5 mg/kg.

### EXEMPLES :

### Exemple 1 : Polymère amphiphile P1

### Synthèse d'un polyglutamate greffé par l'alpha-tocophérol d'origine synthétique

On solubilise 5,5 g d'un alpha-L-polyglutamate (de masse équivalente à environ 10 000 Da) par rapport à un standard en polyoxyéthylène et obtenu par polymérisation de NCAGluOMe suivie d'une hydrolyse comme décrits dans la demande de brevet FR-A-2 801 226) dans 92 ml de diméthylformamide (DMF) en chaulant à 40°C pendant 2 heures. Une fois le polymère solubilisé, on laisse revenir la température à 25 °C et on ajoute successivement 1,49 g de D,L-alpha-tocophérol (> 98 % obtenu de Fluka®) préalablement solubilisé dans 6 ml de DMF, 0,09 g de 4-dimethylaminopyridine préalablement solubilisé dans 6 ml de DMF et 0,57 g de diisopropylcarbodiimide préalablement solubilisé dans 6 ml de DMF. Après 8 heures à 25 °C sous agitation, le milieu réactionnel est versé dans 800 ml d'eau contenant 15 % de chlorure de sodium et d'acide chlorhydrique (pH 2). Le polymère précipité est ensuite récupéré par filtration, lavé par de l'acide chlorhydrique 0,1 N puis par de l'eau. Le polymère est ensuite resolubilisé dans 75 ml de DMF puis reprécipité dans de l'eau contenant comme précédemment du sel et de l'acide à pH 2. Après deux lavages à l'eau, on lave plusieurs fois par de l'éther diisopropylique. Le polymère est ensuite séché à l'étuve sous vide à 40 °C. On obtient un rendement de l'ordre de 85 %.

### Exemple 2 : Polymères amphiphiles P2, P3, P4, P5 et P6

Ces polymères sont obtenus de la même façon que pour l'obtention du polymère P1. Le tableau 1 ci-dessous résume les caractéristiques de ces polymères. Celles du polymère P1 sont données à titre de comparaison.

**TABLEAU 1**

| Polymère | Mn¹ g/mol du polyglutamate | Groupement hydrophobe | % de greffage (RMN)² | Mn¹ g/mol du polymère |
|---|---|---|---|---|
| P1 | 10 000 | alpha-tocophérol³ | 7 | 13 900 |
| P2 | 10 000 | alpha-tocephérol³ | 4 | 14 400 |
| P3 | 16 900 | alpha-tocophérol³ | 4 | 15 200 |
| P4 | 10 000 | Cholestérol | 5 | 11 500 |
| P5 | 16 900 | Cholestérol | 5 | 12 900 |
| P6 | 10 000 | n-Dodécanol | 15 | 11 500 |

| | | | | |
|---|---|---|---|---|
| ¹ En équivalent polyoxyéthylène. ² Taux de greffage molaire estimé par la RMN du proton. ³d'origine synthétique | | | | |

### Exemple 3 : Préparation de 30 ml d'une formulation d'interféron alpha 2b (IFN) à base du polymère P6.

### (a) Préparation d'une solution colloïdale de polymère amphiphile :

On introduit dans un flacon 1,5 g de poudre lyophilisée du polyaminoacide amphiphile P6 de l'exemple 1 ci dessus. Cette poudre est dissoute dans 30 ml d'eau stérile pour injection. La solution de polymère est maintenue 16 heures à 35°C sous agitation magnétique. L'osmolarité de la solution est ajustée à 275 ± 20 mOsmol à l'aide d'un osmomètre Fiske Mark 3, en introduisant la quantité nécessaire d'une solution aqueuse de NaCl 5,13M (30 % p/p). Le pH est ajusté si nécessaire à pH 7,4 ± 0,2 par ajout d'une solution de NaOH 1N. La concentration en polymère est ajustée à 45 mg/ml par ajout d'une solution aqueuse stérile de NaCl 0,15M. La solution de polymère est ensuite filtrée sur un filtre de taille de pore 0,8 et 0,2 microns, puis stockée à 4 °C.

### (b) Association de la protéine au polymère :

Dans un flacon en verre, on mélange ensuite 26,65 ml de la solution colloïdale de polymère P6 précédente et 1,85 ml de solution d'IFN (PC GEN ; solution concentrée à 2,42 mg/ml). L'osmolarité et le pH sont réajustés si nécessaire à 300 ± 20 mOsmol et pH 7, 4 ± 0,2 par ajout de soude 0,1N et chlorure de sodium 0,9 % stérile. La solution chargée en protéine est mise en maturation pendant 5 h à 25°C à l'étuve, puis est ensuite filtrée sur 0,8-0,2 microns. On obtient ainsi 30 ml d'une formulation prête à être injectée contenant 0,15 mg/ml d'IFN et 40 mg/ml de polymère P6.

### Exemple 4: Préparation d'une formulation d'IFN longue action selon la présente invention, à base de l'un des polymères P1 à P5.

La préparation s'effectue comme dans l'exemple 3 en préparant dans un premier temps une solution colloïdale de polymère à 1,25 fois la concentration finale recherchée, puis en mélangeant cette solution avec une solution d'interféron concentrée à 2,42 mg/ml . Le volume de la solution de protéine est déterminé par le choix du rapport de la concentration en polymère, à la concentration en protéine visée. Comme dans l'exemple 3, les ajustements de concentrations et de pH sont réalisés par ajout de solution de NaCl et de soude.

### Exemple 5: Préparation d'une formulation d'InterLeukine-2 (IL2) longue action selon la présente invention à base du polymère P3.

On introduit dans un flacon la quantité de poudre lyophilisée du polymère amphiphile et d'eau stérile nécessaires pour obtenir une concentration en polymère égale à X = 1,3 fois la concentration finale recherchée dans la formulation. La dissolution du polymère est prolongée 16 heurs sous agitation magnétique.
La quantité nécessaire d'IL2 lyophilisée (Prospec) est concentrée à X/(X-1) fois la concentration finale recherchée.
La concentration précise de la solution d' IL2 concentrée est déterminée par dosage UV à 280 nm, à l'aide d'un spectrophotomètre UV Perkin Elmer Lambda 35.
Cette solution d'IL2 est filtrée sur 0,8-0,2 µm et stockée à 4°C. Son pH est ajusté à pH 11 par ajout de NaOH 1 M. On nomme Y le rapport de la concentration en protéine de cette solution à la concentration souhaitée dans la formulation.
On procède ensuite au mélange à température ambiante de la solution de protéine et de la solution de polymère. Pour un volume de polymère, on rajoute X - 1 volumes de solution de protéine. Le pH et l'osmolarité sont ajustés à pH 7,4 ± 0,2 et à 300 ± 20 mOsm.
Ainsi, pour préparer une formulation d'IL2 longue action selon l'invention à base du polymère P3 contenant 20 mg/ml de polymère P3 et 2,5 mg/ml d'IL2, la solution initiale de polymère est concentrée à 26 mg/ml. La solution initiale d'IL2 est concentrée à 11 mg/ml. Pour un volume de polymère, on rajoute 0,3 volumes de solution de protéine.

### Exemple 6: Mesure du diamètre hydrodynamique moyen des nanoparticules de différents polymères PO selon l'invention.

Le diamètre hydrodynamique moyen des particules de polymère PO selon l'invention est mesuré selon le Mode opératoire Md défini ci-après :
Les solutions de PO sont préparées à des concentrations de 1 ou 2 mg/ml en milieu NaCl 0,15M et laissées sous agitation pendant 24 h. Ces solutions sont ensuite filtrées sur 0,8-0,2 µm, avant de les analyser en diffusion dynamique de la lumière grâce à un appareil de type Brookhaven, fonctionnant avec un faisceau laser de longueur d'onde 488 nm et polarisé verticalement. Le diamètre hydrodynamique des nanoparticules de polymère PO est calculé à partir de la fonction d'autocorrélation du champ électrique par la méthode des cumulants, comme décrit dans l'ouvrage « Surfactant Science Series » volume 22, Surfactant Solutions, Ed. R Zana, chap. 3, M. Dekker, 1984.
On obtient les résultats suivants pour les polymères PO P2 P3 P4 et P6 de l'exemple 2 :

**TABLEAU 2**

| Polymère | Diamètre hydrodynamique moyen (nm) |
|---|---|
| P2 | 60 |
| P3 | 90 |
| P4 | 30 |
| P6 | 15 |

### Exemple 7 : Association spontanée d'une protéine aux nanoparticules de polymère PO

Une solution de tampon phosphate à 25 mM est préparée à partir de poudre de NaH₂PO₄ (Sigma refS-0751) et ajustée avec de la soude 1N (SDS ref 3470015) à pH = 7.2.
Une suspension colloïdale de nanoparticules de polymère P1 est préparée par dissolution pendant une nuit du polymère lyophilisé à 5 mg/ml dans la solution de tampon phosphate précédente.
Une solution mère de BSA (Sigma A-2934) est préparée par dissolution pendant deux heures de protéine à 10 mg/ml dans le même tampon.
Les solutions mères ainsi que le tampon sont filtrées sur 0,22 µm.
Des mélanges sont réalisés par ajout de volumes prédéterminés des deux solutions mères et dilution dans le tampon phosphate de façon à avoir au final une gamme d'échantillons ayant une concentration constante en polymère (0,1 mg/ml) et des concentrations croissantes de protéines (0 à 1.8 mg/ml).

Les échantillons sont laissés 5 heures à associer à 25 °C puis ils sont analysés par électrophorèse capillaire dans une méthode dite frontale où il est possible de visualiser séparément la protéine et le complexe protéine - polymère. Pour plus de détails sur cette technique, on consultera l'article suivant : Gao JY, Dublin PL, Muhoberac BB, Anal. Chem. 1997, 69, 2945. Les analyses sont réalisées sur un appareil Agilent G16000A muni d'un capillaire à bulle en silice fondue (type G1600-62-232). La hauteur du premier plateau correspondant à la protéine libre permet de déterminer la concentration en BSA non associée. L'expérience montre que pour des quantités de protéines inférieures à 0,1 g de protéine par g de polymère, la protéine est associée aux nanoparticules de polymère.

### Exemple 8 : Détermination de la concentration de gélification C1 pour les polymères PO P1 à P4 et P6.

Le test GI est appliqué à des formulations d'IFN et d'IL2 associés aux polymères P1 à P6 des exemples 1 et 2. Les concentrations en protéines de ces formulations sont reportées dans le tableau ci-dessous. La mesure du temps de relaxation des formulations en présence de BSA (concentration 30 mg/ml) s'effectue selon le mode opératoire du test GI. La concentration critique C1, pour laquelle le temps de relaxation excède 1 s est reportée sur les tableaux 3 et 4 pour l'IFN et l'IL-2 respectivement:

**TABLEAU 3 :**

| Concentration de gélification induite pour des formulations d'IFN | | | | | |
|---|---|---|---|---|---|
| Polymère | P1 | P2 | P3 | P4 | P6 |
| Concentration en IFN (mg/ml) | 0,3 | 0,15 | 0,15 | 0,15 | 0,3 |
| Concentration C1 (mg/ml) | 17 | >30 | 16 | 17 | >50 |

**TABLEAU 4:**

| Concentration de gélification induite pour des formulations d'IL2 | | | |
|---|---|---|---|
| Polymère | P1 | P3 | P6 |
| Concentration en IL2 (mg/ml) | 2,5 | 2,5 | 2,5 |
| Concentration C1 (mg/ml) | 17 | 17 | > 50 |

### Exemple 9 : Pharmacocinétique de l'IFN chez le chien après injection sous cutanée d'une formulation d'IFN appartenant à la sélection selon l'invention.

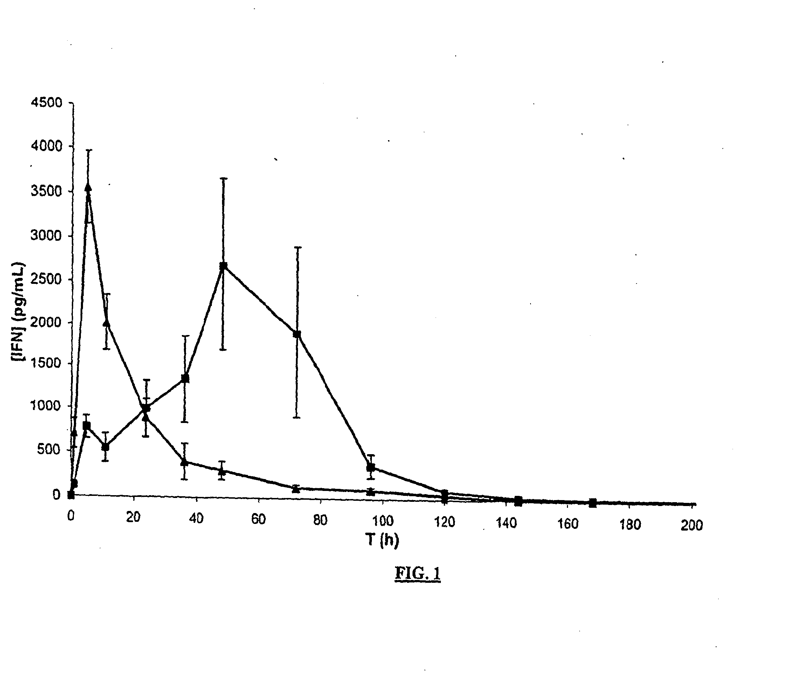

Une formulation (A) d'IFN (concentration 0,3 mg/ml) et de polymère amphiphile P1 à la concentration de 30 mg/ml est préparée selon le mode opératoire décrit dans l'exemple 3.
Cette formulation est injectée par voie sous cutanée à des chiens Beagles (n= 3), à la dose de 60 µg / kg). Des prélèvements de sérum sont effectués aux temps 1, 5, 11, 24, 36, 48, 72, 96, 120, 144, 168 et 240 heures. La concentration plasmatique en IFN est mesurée sur ces prélèvements par dosage ELISA (kit immunotech IM 3193).
On obtient ainsi un profil de concentration plasmatique moyen tel que représenté sur la figure 1 qui met clairement en évidence la libération prolongée de la protéine dans le sérum par rapport à une formulation témoin (D) hors invention d'IFN (concentration 0,3 mg/ml) et de polymère amphiphile P6 à la concentration de 40 mg/ml (cf. tableau 5, exemple 10). Sur un plan quantitatif, la prolongation de la libération de l'IFN par les formulations selon l'invention est estimée par la mesure :
(a) du temps Tmax, médiane du temps pour lequel la concentration plasmatique est maximale,
(b) du temps T50, moyenne du temps au bout duquel l'aire sous la courbe de concentration plasmatique atteint 50 % de sa valeur maximale mesurée.
Dans le cas de cette formulation, les temps Tmax et T50 prennent les valeurs :

| | |
|---|---|
| Tmax = | 48 heures |
| T50 = | 54,2 heures |

### Exemple 10 : Pharmacocinétique de l'IFN chez le chien après injection sous cutanée de diverses formulations d'IFN à base de polyaminoacides amphiphiles.

Les formulations suivantes sont préparées selon le mode opératoire décrit dans l'exemple 3.

**TABLEAU 5**

| Formulation | Polymère | Concentration en polymère (mg/ml) | Concentration en IFN (mg/ml) | Temps de relaxation Tr(s) |
|---|---|---|---|---|
| A | P1 | 30 | 0,3 | > 10 |
| B | P1 | 10,5 | 0,15 | < 0,3 |
| C | P2 | 15 | 0,15 | < 0,03 |
| D | P6 | 40 | 0,3 | 0,4 |

La formulation A a une concentration en polymère supérieure à la concentration de gélification C1 mesurée dans l'exemple 8. En d'autres termes, le temps de relaxation, mesuré dans le test GI est supérieur à 1 seconde. Cette formulation A appartient donc à la sélection selon l'invention. En revanche, les formulations B, C et D ont des concentrations inférieures à leurs concentrations de gélification et n'appartiennent pas à la sélection selon l'invention.

Ces formulations sont injectées à la dose de 60 µg/ kg à des chiens Beagles. Des prélèvements de plasma sont effectués aux temps 1, 5, 11, 24, 36, 48, 72, 96, 120, 144, 168 et 240 heures. La concentration plasmatique en IFN est mesurée comme dans l'exemple précédent.
Les temps Tmax et T50 pour les formulations A, B, C et D sont reportés dans le tableau 6 ci-dessous.

**TABLEAU 6**

| Référence formulation | Tmax (h) | T50 (h) |
|---|---|---|
| A | 48 | 54,2 |
| B | 5 | 16,7 |
| C | 11 | 19,3 |
| D | 5 | 17,3 |

Ainsi, la formulation A, qui appartient à la sélection selon l'invention, présente une durée de libération considérablement accrue par rapport aux formulations B, C, et D qui n'appartiennent pas à la sélection selon l'invention.

### Exemple 11 : Pharmacocinétique de l'interleukine 2 (IL2) chez le singe après injection sous cutanée de diverses formulations à base de polyaminoacides amphiphiles.

Les formulations suivantes sont préparées selon le mode opératoire décrit dans l'exemple 5.

**TABLEAU 7**

| Référence | Polymère | Concentration en polymère (mg/ml) | Concentration en IL2 (mg/ml) |
|---|---|---|---|
| E | P1 | 30 | 2,5 |
| F | P3 | 20 | 2,5 |
| G | P6 | 40 | 2,5 |

Les formulations E et F, qui ont une concentration en polymère supérieure à la concentration de gélification C1 mesurée dans l'exemple 8 appartiennent donc à la sélection selon l'invention. En revanche, la formulation G a une concentration inférieure à la concentration de gélification C1 et n'appartient pas à la sélection selon l'invention.
Ces formulations sont injectées à la dose 0,5 mg/kg à des singes Cynomolgus. Des prélèvements de plasma sont effectués aux temps 1, 5, 11, 24, 36, 48, 72, 96, 120, 144, 168 et 240 heures. La concentration plasmatique en IL2 est mesurée sur ces prélèvements par dosage ELISA (kit Immunotech IM 3583).

Les temps Tmax et T50 pour les formulations E, F et G sont reportés dans le tableau 8 ci-dessous.

**TABLEAU 8**

| Référence formulation | Tmax (h) | T50 (h) |
|---|---|---|
| E | 32 | 34,5 |
| F | 32 | 37,5 |
| G | 4 | 10,5 |

Ainsi, les formulations E et F, qui appartiennent à la sélection selon l'invention, présentent une durée de libération considérablement accrue par rapport à la formulation G qui n'appartient pas à la sélection selon l'invention.

### Exemple 12 : observation de la gélification in vivo des formulations selon l'invention après injection sous-cutanée.

Le comportement sous cutané des formulations selon l'invention a été étudié chez le porc domestique. On a procédé à des injections sous la peau du ventre, à 4 mm de profondeur, de six porcs domestiques, avec 0,3 ml des formulations suivantes :
Formulation A : solution aqueuse isotonique à pH 7,3 du polymère P6 de l'exemple 2 concentré à 45 mg/ml.
Formulation B : solution aqueuse isotonique à pH 7,3 du polymère P1 de l'exemple 1 concentré à 20 mg/ml.
Les sites injectés ont été prélevés 72 heures après administration. L'examen histologique révèle la présence d'un dépôt gélifié de polymère pour la formulation B. Il se présente sous forme de plages uniformément colorées. Ce phénomène n'est en revanche pas observé pour la formulation A pour laquelle le polymère est infiltré entre les fibres de collagène.
On peut souligner que la matrice de polymère B est parfaitement bio dégradable car le tissu est complètement revenu à son état normal après 21 jours.

## Revendications

1. Formulation pharmaceutique liquide pour la libération prolongée de principe(s) actif(s) -PA-, cette formulation comprenant au moins un principe actif PA, de préférence en solution aqueuse, et une suspension colloïdale, aqueuse, de basse viscosité, à base de particules submicroniques de polymère (PO) biodégradable, hydrosoluble et porteur de groupements hydrophobes (GH), lesdites particules étant associées de façon non covalente avec ledit principe actif (PA),
**caractérisée :**
**◆ en ce que** le milieu dispersif de la suspension est essentiellement constitué par de l'eau,
◆ en ce que sa concentration en [PO] est fixée à une valeur suffisamment élevée de sorte que ladite formulation pharmaceutique est apte à être injectée par voie parentérale et à former ensuite in vivo un dépôt gélifié, cette formation de dépôt gélifié :
o étant, d'une part, au moins en partie provoquée par au moins une protéine physiologique présente in vivo,
o et permettant, d'autre part, de prolonger et de contrôler la durée de libération du PA in vivo, au-delà de 24 h après l'administration,
◆ en ce qu'elle est liquide dans les conditions d'injection,
◆ et en ce qu'elle est également liquide à la température et/ou au pH physiologiques, et/ou en présence :
***** d'électrolyte physiologique en concentration physiologique,
***** et/ou d'au moins un tensioactif.

2. Formulation pharmaceutique liquide pour la libération prolongée de principe(s) actif(s) -PA-, cette formulation :
o étant liquide en atmosphère ambiante,
o étant également liquide à la température et/ou au pH physiologiques et/ou en présence :
* d'électrolyte physiologique en concentration physiologique,
* et/ou d'au moins un tensioactif,
o et comprenant au moins un principe actif PA, de préférence en solution aqueuse, et une suspension colloïdale, aqueuse, de basse viscosité, à base de particules submicroniques de polymère PO biodégradable, hydrosoluble et porteur de groupements hydrophobes GH, lesdites particules étant associées de façon non covalente avec ledit principe actif PA et le milieu dispersif de la suspension étant essentiellement constitué par de l'eau,
**caractérisée en ce que** sa concentration en [PO] est fixée à une valeur suffisamment élevée pour permettre la formation de dépôt gélifié in vitro, en présence d'au moins une protéine.

3. Formulation selon l'une quelconque des revendications précédentes, **caractérisée en ce que** sa concentration en [PO] est telle que:
■ [PO] ≥ 0,9.C1,
■ de préférence 20.C1 ≥ [PO] ≥ C1,
■ et mieux encore 10.C1 ≥ [PO] ≥ C1
avec C1 représentant la concentration de "*gélification induite"* des particules de PO telle que mesurée dans un test GI.

4. Formulation selon l'une quelconque des revendications précédentes, **caractérisée en ce que** sa viscosité est inférieure ou égale à 5 Pa.s.

5. Formulation selon l'une quelconque des revendications précédentes, **caractérisée en ce que** les polymères modifiés hydrophobes PO sont sélectionnés dans le groupe comprenant: les polyaminoacides, les polysaccharides de préférence dans le sous-groupe comprenant les pullulanes et/ou les chitosans et/ou les mucopolysaccharides, les gélatines ou leurs mélanges.

6. Formulation selon l'une quelconque des revendications précédentes, **caractérisée en ce que** les groupements hydrophobes (GH) se situent latéralement à la chaîne.

7. Formulation selon l'une quelconque des revendications précédentes, **caractérisée en ce que** le polymère (PO) est un polyaminoacide formé par des unités acide aspartique et/ou des unités acide glutamique, au moins une partie de ces unités étant porteuses de greffons comportant au moins un groupement hydrophobe (GH).

8. Formulation selon la revendication 7, **caractérisée en ce que** le (ou les) PO est ou (sont) définis par la formule générale (I) suivante : 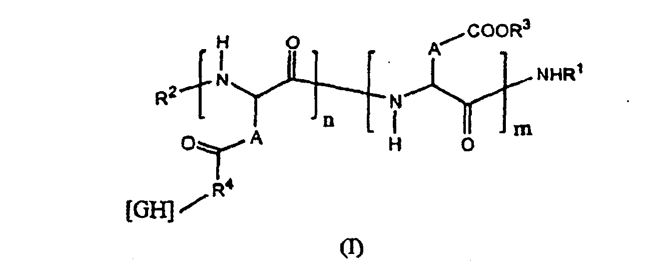 dans laquelle :
■ R¹ représente un H, un alkyle linéaire en C2 à C10 ou ramifié en C3 à C10, un benzyle, une unité acide aminé terminale ou -R⁴-[GH] ;
■ R² représente un H, un groupe acyle linéaire en C2 à C10 ou ramifié en C3 à C10, un pyroglutamate ou -R⁴-[GH] ;
■ R³ est un H ou une entité cationique, de préférence sélectionnée dans le groupe comprenant :
- les cations métalliques avantageusement choisis dans le sous-groupe comprenant : le sodium, le potassium, le calcium, le magnésium,
- les cations organiques avantageusement choisis dans le sous-groupe comprenant:
• les cations à base d'amine,
• les cations à base d'oligoamine,
• les cations à base de polyamine (la polyethylèneimine étant particulièrement préférée),
• les cations à base d'acide(s) aminé(s) avantageusement choisis dans la classe comprenant les cations à base de lysine ou d'arginine,
- ou les polyaminoacides cationiques avantageusement choisis dans le sous-groupe comprenant la polylysine ou l'oligolysine ;
■ R⁴ représente une liaison directe ou un "espaceur" à base de 1 à 4 unités acide aminé ;
■ A représente indépendamment un radical -CH₂- (unité acide aspartique) ou-CH₂-CH₂- (unité acide glutamique) ;
■ n/(n+m) est défini comme le taux de greffage molaire et sa valeur est suffisamment basse pour que PO mis en solution dans l'eau à pH 7 et à 25 °C, forme une suspension colloïdale de particules submicroniques de PO, de préférence n/(n + m) est compris entre 1 à 25 % molaire et mieux encore entre 1 et 15 % molaire;
■ n + m varie de 10 à 1000, de préférence entre 50 et 300 ;
■ GH représente un groupement hydrophobe.

9. Formulation selon la revendication 7, **caractérisée en ce que** le (ou les) PO répond (ent) à l'une des formules générales (II), (III) et (IV) suivantes : 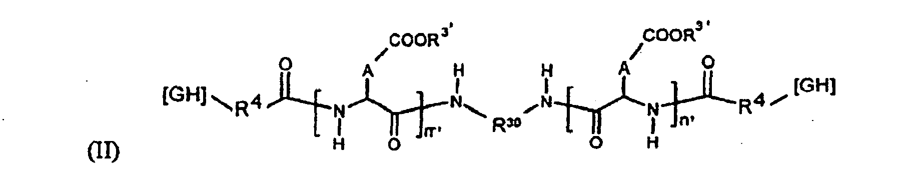 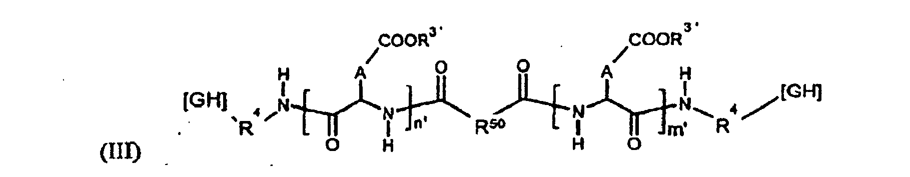 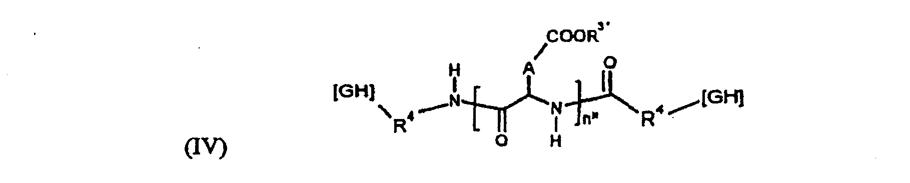 dans lesquelles :
■ GH représente un groupement hydrophobe ;
■ R³⁰ est un groupement alkyle linéaire en C2 à C6 ;
■ R^{3'} est un H ou une entité cationique, de préférence sélectionnée dans le groupe comprenant :
- les cations métalliques avantageusement choisis dans le sous-groupe comprenant : le sodium, le potassium, le calcium, le magnésium,
- les cations organiques avantageusement choisis dans le sous-groupe comprenant :
• les cations à base d'amine,
• les cations à base d'oligoamine,
• les cations à base de polyamine (la polyéthylèneimine étant particulièrement préférée),
• les cations à base d'acide(s) aminé(s) avantageusement choisis dans la classe comprenant les cations à base de lysine ou d'arginine,
- ou les polyaminoacides cationiques avantageusement choisis dans le sous-groupe comprenant la polylysine ou l'oligolysine,
■ R⁵⁰ est un groupement alkyle, dialcoxy ou diamine en C2 à C6 ;
■ R⁴ représente une liaison directe ou un "espaceur" à base de 1 à 4 unités acide aminé ;
■ A représente indépendamment un radical -CH₂- (unité acide aspartique) ou-CH₂-CH₂- (unité acide glutamique) ;
■ n' + m' ou n"est défini comme le degré de polymérisation et varie de 10 à 1000, de préférence entre 50 et 300.

10. Formulation selon la revendication 8 ou 9, **caractérisée en ce que** les groupements GH du PO représentent chacun indépendamment les uns des autres un radical monovalent de formule suivante : 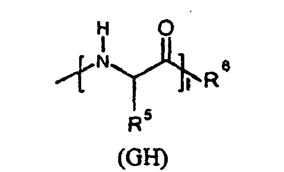 dans laquelle :
- R⁵ représente un méthyle (alanine), isopropyle (valine), isobutyle (leucine), secbutyle (isoleucine), benzyle (phénylalanine) ;
- R⁶ représente un radical hydrophobe comportant de 6 à 30 atomes de carbone;
- 1 varie de 0 à 6.

11. Formulation selon la revendication 10, **caractérisée en ce que** tout ou partie des radicaux hydrophobes R⁶ des PO sont choisis de façon indépendante dans le groupe de radicaux comportant :
■ un alcoxy linéaire ou ramifié comportant de 6 à 30 atomes de carbone et pouvant comporter au moins un hétéroatome (de préférence O et/ou N et/ou S) et/ou au moins une insaturation,
■ un alcoxy comportant 6 à 30 atomes de carbone et ayant un ou plusieurs carbocycles annelés et contenant éventuellement au moins une insaturation et/ou au moins un hétéroatome (de préférence O et/ou N et/ou S),
■ un alcoxyaryle ou un aryloxyalkyle de 7 à 30 atomes de carbone et pouvant comporter au moins une insaturation et/ou au moins un hétéroatome (de préférence O et/ou N et/ou S).

12. Formulation selon la revendication 10 ou 11 **caractérisée en ce que** le radical hydrophobe R⁶ du greffon du PO est issu d'un précurseur alcoolique choisi dans le groupe comprenant: l'octanol, le dodécanol, le tétradécanol, l'hexadécanol, l'octadécanol, l'oleylalcool, le tocophérol ou le cholestérol.

13. Formulation selon la revendication 7, **caractérisée en ce que** le PO est constitué d'un homopolymère d'alpha-L-glutamate ou d'acide alpha-L-glutamique.

14. Formulation selon la revendication 7, **caractérisée en ce que** le PO est constitué d'un homopolymère d'alpha-L-aspartate ou d'acide alpha-L-aspartique.

15. Formulation selon la revendication 7, **caractérisée en ce que** le PO est constitué d'un copolymère d'alpha-L-aspartate/alpha-L-glutamate ou d'acide alpha-L-aspartique/alpha-L-glutamique.

16. Formulation selon la revendication 15, **caractérisée en ce que** dans le PO, la distribution des unités acide aspartique et/ou acide glutamique porteuses de greffons comportant au moins un motif GH est telle que le polymère ainsi constitué est soit aléatoire, soit de type bloc, soit de type multibloc.

17. Formulation selon la revendication 1, **caractérisée en ce que** la masse molaire du PO se situe entre 2000 et 100 000 g/mole, et de préférence entre 5000 et 40 000 g/mole.

18. Formulation selon la revendication 7, **caractérisée en ce que** le PO est porteur d'au moins un greffon de type polyalkylène glycol lié à une unité glutamate et/ou aspartate.

19. Formulation selon la revendication 18, **caractérisée en ce que** le greffon de type polyalkylène glycol est de formule (V) suivante : 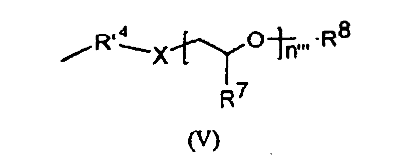 dans laquelle :
- R'⁴ représente une liaison directe ou un "espaceur" à base de 1 à 4 unités acide aminé ;
- X est un hétéroatome choisi dans le groupe comportant l'oxygène, l'azote ou un soufre ;
- R⁷ et R⁸ représentent indépendamment un H, un alkyle linéaire en C1 à C4 ;
- n"' varie de 10 à 1000, de préférence de 50 à 300.

20. Formulation selon la revendication 18 ou 19, **caractérisée en ce que** le polyalkylèneglycol est un polyethylène glycol.

21. Formulation selon l'une quelconque des revendications 18 à 20, **caractérisée en ce que** le pourcentage molaire de greffage du polyalkylène glycol varie de 1 à 30 %.

22. Formulation selon l'une quelconque des revendications précédentes, **caractérisée en ce que** le PA est une protéine, une glycoprotéine, une protéine liée à une ou plusieurs chaînes polyalkylèneglycol [de préférence polyéthylèneglycol (PEG) : "protéine-PEGylée"], un polysaccharide, un liposaccharide, un oligonucléotide, un polynucléotide ou un peptide,
de préférence sélectionné parmi les hémoglobines, les cytochromes, les albumines, les interférons, les cytokines, les antigènes, les anticorps, l'érythropoïétine, l'insuline, les hormones de croissance, les facteurs VIII et IX, les interleukines ou leurs mélanges, les facteurs stimulants de l'hématopoïèse.

23. Formulation selon l'une quelconque des revendications 1 à 21, **caractérisée en ce que** le principe actif est une "petite" molécule organique hydrophobe, hydrophile ou amphiphile.

24. Formulation selon l'une quelconque des revendications 1 à 22, **caractérisée en ce que** sa fraction massique en PA non associé aux particules submicroniques [PA non associé] en % en poids est telle que :
o [PA non-associé] ≤ 1
o de préférence [PA non-associé] ≤ 0,5.

25. Formulation selon l'une quelconque des revendications précédentes, **caractérisée en ce qu'**elle est injectable par voie parentérale, sous-cutanée, intramusculaire, intradermique, intrapéritonéale, intracérébrale ou dans une tumeur.

26. Formulation selon l'une quelconque des revendications précédentes, **caractérisée en ce qu'**elle est destinée à la préparation de médicaments, en particulier pour administration parentérale, sous-cutanée, intramusculaire, intradermique, intrapéritonéale, intracérébrale ou dans une tumeur, voire par voie orale, nasale, vaginale ou oculaire.

27. Procédé de préparation de médicaments, en particulier pour administration parentérale, sous-cutanée, intramusculaire, intradermique, intrapéritonéale, intracérébrale ou dans une tumeur, voire par voie orale, nasale, vaginale ou oculaire, **caractérisé en ce qu'**il consiste essentiellement à mettre en oeuvre au moins une formulation selon l'une quelconque des revendications 1 à 26.

28. Produit dérivé **caractérisé en ce qu'**il comprend des particules submicroniques, formées par des associations non covalentes PO/PA telles que définies dans la revendication 1 et **en ce qu'**il est obtenu à partir de la formulation selon l'une quelconque des revendications 1 à 26.

29. Produit dérivé selon la revendication 28, **caractérisé en ce qu'**il est constitué par une poudre ou par un gel.

30. Procédé de préparation de la formulation selon l'une quelconque des revendications 1 à 26, **caractérisé en ce qu'**il consiste essentiellement :
◆ à mettre en oeuvre une suspension colloïdale de nanoparticules d'au moins un PO,
◆ à mélanger cette suspension colloïdale de nanoparticules de PO avec au moins un PA, de préférence en solution aqueuse,
◆ à ajouter éventuellement au moins un excipient,
◆ au besoin à ajuster le pH et/ou l'osmolarité et,
◆ éventuellement à filtrer la suspension ainsi obtenue.

31. Procédé selon la revendication 30, **caractérisé en ce que** le (ou les) PA(s) est (sont) sous forme de suspension ou de solution aqueuse pour le mélange avec la suspension colloïdale de nanoparticules de PO.

32. Procédé de préparation de la formulation selon l'une quelconque des revendications 1 à 26, **caractérisé en ce qu'**il consiste essentiellement:
à mettre en oeuvre une poudre de nanoparticules d'au moins un PO,
à mélanger cette poudre avec une suspension ou solution aqueuse d'au moins un PA, de préférence en solution aqueuse,
à ajouter éventuellement au moins un excipient,
au besoin à ajuster le pH et/ou l'osmolarité et,
éventuellement à filtrer la suspension ainsi obtenue.

33. Procédé de préparation de la formulation selon l'une quelconque des revendications 1 à 26, **caractérisé en ce qu'**il consiste essentiellement :
à mettre en oeuvre une poudre issue du séchage de la formulation liquide selon l'une quelconque des revendications 1 à 26,
à mélanger cette poudre avec un milieu liquide aqueux, de préférence sous agitation,
à ajouter éventuellement au moins un excipient,
au besoin à ajuster le pH et/ou l'osmolarité et,
éventuellement à filtrer la suspension ainsi obtenue.

34. Procédé de préparation d'une poudre dérivée de la formulation selon l'une quelconque des revendications 1 à 26, **caractérisé en ce que** ladite poudre est obtenue par séchage de la formulation selon l'une quelconque des revendications 1 à 26.

## Claims

1. Liquid pharmaceutical formulation for the sustained release of active ingredient(s) -AI-, this formulation comprising at least one active ingredient AI, preferably in aqueous solution, and a low-viscosity aqueous colloidal suspension based on biodegradable water-soluble submicronic polymer (PO) particles bearing hydrophobic groups (GH), said particles being bound in a non-covalent manner to said active ingredient (AI),
**characterized:**
**◆ in that** the dispersive medium of the suspension is essentially constituted by water,
◆ in that its [PO] concentration is fixed at a sufficiently high value such that said pharmaceutical formulation can be injected by parenteral route and then form a gelled deposit *in vivo*, the formation of this gelled deposit:
○ on the one hand, being at least partly induced by at least one physiological protein present *in vivo,*
○ and, on the other hand, allowing the duration of the *in vivo* release of the AI to be sustained and controlled beyond 24 h after the administration,
◆ in that it is liquid under injection conditions,
◆ and in that it is also liquid at physiological temperature and/or pH, and/or in the presence of:
* physiological electrolyte in a physiological concentration,
* and/or at least one surfactant.

2. Liquid pharmaceutical formulation for the sustained release of active ingredient(s) -AI-, this formulation:
o being liquid in ambient atmosphere,
o also being liquid at physiological temperature and/or pH, and/or in the presence of:
* physiological electrolyte in a physiological concentration,
* and/or at least one surfactant.
o and comprising at least one active ingredient AI, preferably in aqueous solution, and a low-viscosity aqueous colloidal suspension based on biodegradable water-soluble submicronic polymer PO particles bearing hydrophobic groups GH, said particles being bound in a non-covalent manner to said active ingredient AI and the dispersive medium of the suspension being essentially constituted by water,
**characterized in that** its [PO] concentration is fixed at a sufficiently high value to allow the *in vitro* formation of a gelled deposit, in the presence of at least one protein.

3. Formulation according to any one of the previous claims, **characterized in that** its [PO] concentration is such that:
■ [PO] ≥ 0.9.C1,
■ preferably 20.C1 ≥ [PO] ≥ C1,
■ and even better 10.C1 ≥ [PO] ≥ C1
with C1 representing the concentration of "*induced gelling*" of the PO particles as measured in an IG test.

4. Formulation according to any one of the previous claims, **characterized in that** its viscosity is less than or equal to 5 Pa.s.

5. Formulation according to any one of the previous claims, **characterized in that** the modified hydrophobic polymers PO are selected from the group comprising: polyamino acids, polysaccharides preferably from the subgroup comprising pullulans and/or chitosans and/or mucopolysaccharides, gelatins or mixtures thereof.

6. Formulation according to any one of the previous claims, **characterized in that** the hydrophobic groups (GH) are situated laterally to the chain.

7. Formulation according to any one of the previous claims, **characterized in that** the polymer (PO) is a polyamino acid formed by aspartic acid units and/or glutamic acid units, at least some of these units bearing grafts comprising of at least one hydrophobic group (GH).

8. Formulation according to claim 7, **characterized in that** the PO(s) is (are) defined by the following general formula (I): 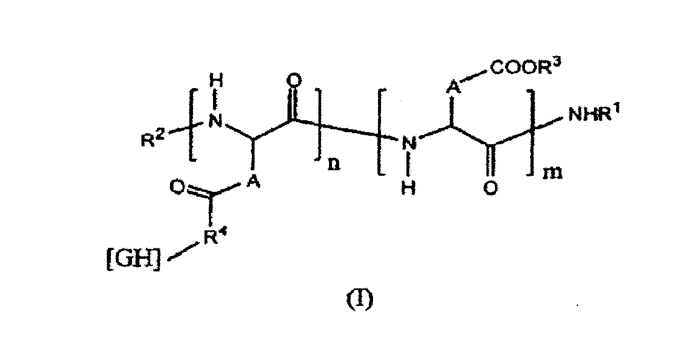 in which:
■ R¹ represents an H, a linear C2 to C10 or branched C3 to C10 alkyl, a benzyl, a terminal amino acid unit or -R⁴-[GH];
■ R² represents an H, a linear C2 to C10 or branched C3 to C10 acyl group, a pyroglutamate or -R⁴-[GH];
■ R³ is an H or a cationic entity, preferably selected from the group comprising:
- metallic cations advantageously chosen from the subgroup comprising: sodium, potassium, calcium, magnesium,
- organic cations advantageously chosen from the subgroup comprising:
• cations based on amine,
• cations based on oligoamine,
• cations based on polyamine (polyethyleneimine being particularly preferred),
• cations based on amino acid(s) advantageously chosen from the class comprising cations based on lysine or arginine,
- or cationic polyamino acids advantageously chosen from the subgroup comprising polylysine or oligolysine;
■ R⁴ represents a direct bond or a "spacer" based on 1 to 4 amino acid units;
■ A represents independently a -CH₂- (aspartic acid unit) or -CH₂-CH₂-(glutamic acid unit) radical;
■ n/(n+m) is defined as the molar grafting rate and its value is sufficiently low that PO solubilized in water at pH 7 and 25°C forms a colloidal suspension of submicronic PO particles, preferably n/(n + m) is comprised between 1 and 25 molar % and even better between 1 and 15 molar %;
■ n + m varies from 10 to 1,000, preferably between 50 and 300;
■ GH represents a hydrophobic group.

9. Formulation according to claim 7, **characterized in that** the PO(s) corresponds (correspond) to one of the following general formulae (II), (III) and (IV): 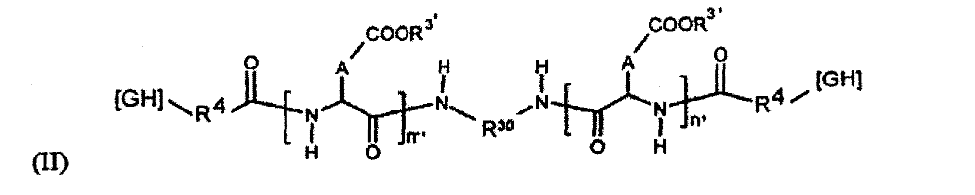 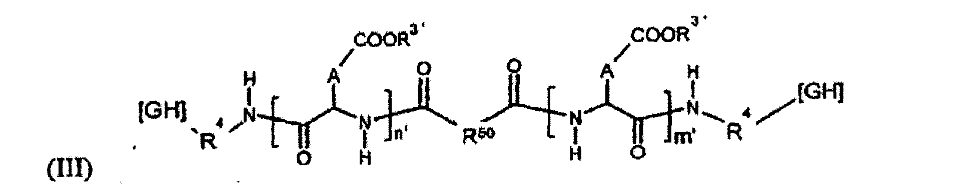 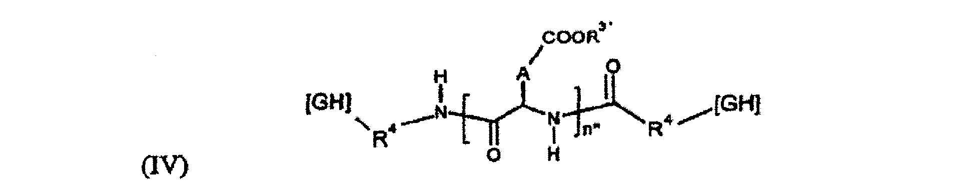 in which:
■ GH represents a hydrophobic group;
■ R³⁰ is a linear C2 to C6 alkyl group;
■ R^{3'} is an H or a cationic entity, preferably selected from the group comprising:
- metallic cations advantageously chosen from the subgroup comprising: sodium, potassium, calcium, magnesium,
- organic cations advantageously chosen from the subgroup comprising:
• cations based on amine,
• cations based on oligoamine,
• cations based on polyamine (polyethyleneimine being particularly preferred),
• cations based on amino acid(s) advantageously chosen from the class comprising cations based on lysine or arginine,
- or cationic polyamino acids advantageously chosen from the subgroup comprising polylysine or oligolysine;
■ R⁵⁰ is a C2 to C6 alkyl, dialkoxy or diamine group;
■ R⁴ represents a direct bond or a "spacer" based on 1 to 4 amino acid units;
■ A represents independently a -CH₂- (aspartic acid unit) or -CH₂-CH₂-(glutamic acid unit) radical;
■ (n' + m') or n" is defined as the degree of polymerization and varies from 10 to 1,000, preferably between 50 and 300.

10. Formulation according to claim 8 or 9, **characterized in that** the GH groups of the PO each represent independently of each other a monovalent radical of the following formula: 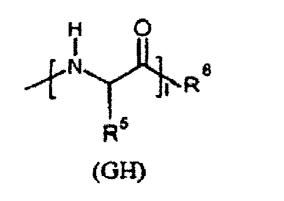 in which:
- R⁵ represents a methyl (alanine), isopropyl (valine), isobutyl (leucine), sec-butyl (isoleucine), benzyl (phenylalanine);
- R⁶ represents a hydrophobic radical comprising 6 to 30 carbon atoms;
- 1 varies from 0 to 6.

11. Formulation according to claim 10, **characterized in that** all or some of the hydrophobic radicals R⁶ of the PO are chosen independently from the group of radicals comprising:
■ a linear or branched alkoxy comprising 6 to 30 carbon atoms and can comprise at least one heteroatom (preferably O and/or N and/or S) and/or at least one unsaturation,
■ an alkoxy comprising 6 to 30 carbon atoms and having one or more ring carbocycles and optionally contains at least one unsaturation and/or at least one heteroatom (preferably O and/or N and/or S),
■ an alkoxyaryl or an aryloxyalkyl of 7 to 30 carbon atoms which can comprise at least one unsaturation and/or at least one heteroatom (preferably O and/or N and/or S).

12. Formulation according to claim 10 or 11, **characterized in that** the hydrophobic radical R⁶ of the graft of the PO results from an alcoholic precursor chosen from the group comprising: octanol, dodecanol, tetradecanol, hexadecanol, octadecanol, oleyl alcohol, tocopherol or cholesterol.

13. Formulation according to claim 7, **characterized in that** the PO is constituted by an alpha-L-glutamate or alpha-L-glutamic acid homopolymer.

14. Formulation according to claim 7, **characterized in that** the PO is constituted by an alpha-L-aspartate or alpha-L-aspartic acid homopolymer.

15. Formulation according to claim 7, **characterized in that** the PO is constituted by an alpha-L-aspartate/alpha-L-glutamate or alpha-L-aspartic acid/alpha-L-glutamic acid copolymer.

16. Formulation according to claim 15, **characterized in that,** in the PO, the distribution of the aspartic acid and/or glutamic acid units bearing grafts comprising at least one GH unit is such that the polymer thus constituted is either random, or of block type, or of multiblock type.

17. Formulation according to claim 1, **characterized in that** the molar mass of the PO is situated between 2,000 and 100,000 g/mole, and preferably between 5,000 and 40,000 g/mole.

18. Formulation according to claim 7, **characterized in that** the PO bears at least one graft of polyalkylene glycol type linked to a glutamate and/or aspartate unit.

19. Formulation according to claim 18, **characterized in that** the graft of polyalkylene glycol type has the following formula (V): 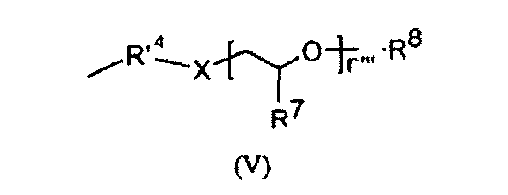 in which:
- R'⁴ represents a direct bond or a "spacer" based on 1 to 4 amino acid units;
- X is a heteroatom chosen from the group comprising oxygen, nitrogen or a sulphur;
- R⁷ and R⁸ represent independently an H, a linear C 1 to C4 alkyl;
- n"' varies from 10 to 1,000, preferably from 50 to 300.

20. Formulation according to claim 18 or 19, **characterized in that** the polyalkylene glycol is a polyethylene glycol.

21. Formulation according to any one of claims 18 to 20, **characterized in that** the molar grafting percentage of the polyalkylene glycol varies from 1 to 30%.

22. Formulation according to any one of the previous claims, **characterized in that** the AI is a protein, a glycoprotein, a protein linked to one or more polyalkylene glycol chains [preferably polyethylene glycol (PEG): "PEGylated protein"], a polysaccharide, a liposaccharidc, an oligonucleotide, a polynucleotide or a peptide, preferably selected from the haemoglobins, cytochromes, albumins, interferons, cytokines, antigens, antibodies, erythropoietin, insulin, growth hormones, factors VIII and IX, the interleukins or mixtures thereof, haematopoiesis-stimulating factors.

23. Formulation according to any one of claims 1 to 21, **characterized in that** the active ingredient is a "small" hydrophobic, hydrophilic or amphiphilic organic molecule.

24. Formulation according to any one of claims 1 to 22, **characterized in that** its fraction by mass of AI not bound to the submicronic particles [non-bound AI] in % by weight is such that:
o [non-bound AI] ≤ 1
o preferably [non-bound AI] ≤ 0.5.

25. Formulation according to any one of the previous claims, **characterized in that** it can be injected by parenteral, subcutaneous, intramuscular, intradermal, intraperitoneal or intracerebral route or into a tumour.

26. Formulation according to any one of the previous claims, **characterized in that** it is intended for the preparation of medicaments, in particular for parenteral, subcutaneous, intramuscular, intradermal, intraperitoneal or intracerebral administration or administration into a tumour, or even by oral, nasal, vaginal or ocular route.

27. Method for the preparation of medicaments, in particular for parenteral, subcutaneous, intramuscular, intradermal, intraperitoneal or intracerebral administration or administration into a tumour, or even by oral, nasal, vaginal or ocular route, **characterized in that** it consists essentially of utilizing at least one formulation according to any one of claims 1 to 26.

28. Derived product, **characterized in that** it comprises submicronic particles, formed by non-covalent PO/AI associations as defined in claim 1 and **in that** it is obtained from the formulation according to any one of claims 1 to 26.

29. Derived product according to claim 28, **characterized in that** it is constituted by a powder or by a gel.

30. Method for the preparation of the formulation according to any one of claims 1 to 26, **characterized in that** it essentially consists of:
◆ utilizing a colloidal suspension of nanoparticles of at least one PO,
◆ mixing this colloidal suspension of PO nanoparticles with at least one AI, preferably in aqueous solution,
◆ optionally adding at least one excipient,
◆ adjusting the pH and/or the osmolarity as required, and
◆ optionally filtering the suspension thus obtained.

31. Method according to claim 30, **characterized in that** the AI(s) is (are) in the form of an aqueous suspension or solution for mixture with the colloidal suspension of PO nanoparticles.

32. Method for the preparation of the formulation according to any one of claims 1 to 26, **characterized in that** it essentially consists of:
utilizing a powder of nanoparticles of at least one PO,
mixing this powder with an aqueous suspension or solution of at least one AI, preferably in aqueous solution,
optionally adding at least one excipient,
adjusting the pH and/or the osmolarity as required, and
optionally filtering the suspension thus obtained.

33. Method for the preparation of the formulation according to any one of claims 1 to 26, **characterized in that** it essentially consists of:
utilizing a powder resulting from the drying of the liquid formulation according to any one of claims 1 to 26,
mixing this powder with an aqueous liquid medium, preferably under stirring,
optionally adding at least one excipient,
adjusting the pH and/or osmolarity as required, and
optionally filtering the suspension thus obtained.

34. Method for the preparation of a powder derived from the formulation according to any one of claims 1 to 26, **characterized in that** said powder is obtained by drying the formulation according to any one of claims 1 to 26.

## Patentansprüche

1. Flüssige pharmazeutische Formulierung zur verzögerten Freisetzung von Wirkstoff(en) - PA -, wobei diese Formulierung mindestens einen Wirkstoff PA, bevorzugt in wässriger Lösung, und eine wässrige kolloidale Suspension mit geringer Viskosität umfasst, die auf Submikronpartikeln aus wasserlöslichem, biologisch abbaubarem Polymer (PO) basiert, das hydrophobe Gruppen (GH) trägt, wobei die Partikeln nicht auf kovalente Art mit dem Wirkstoff (PA) assoziiert sind,
**dadurch gekennzeichnet:**
**◆ dass** das Dispersionsmedium der Suspension im Wesentlichen aus Wasser besteht,
◆ dass ihre [PO]-Konzentration auf einen ausreichend hohen Wert festgelegt ist, derart, dass die pharmazeutische Formulierung auf parenteralem Weg injiziert werden und anschließend in vivo eine gelatinierte Ablagerung bilden kann, wobei die Bildung einer gelatinierten Ablagerung:
o einerseits mindestens zum Teil durch mindestens ein physiologisches Protein hervorgerufen wird, das in vivo vorhanden ist,
o und andererseits ermöglicht, die Dauer der Freisetzung des PA in vivo über 24 Std. nach der Verabreichung hinaus zu verlängern und zu kontrollieren,
◆ dass sie unter den Injektionsbedingungen flüssig ist,
◆ und dass sie bei der Temperatur und/oder den physiologischen pH-Werten, und/oder in Gegenwart:
* eines physiologischen Elektrolyts in physiologischer Konzentration,
* und/oder mindestens eines oberflächenaktiven Stoffs
ebenfalls flüssig ist.

2. Flüssige pharmazeutische Formulierung zur verzögerten Freisetzung von Wirkstoff(en) - PA -, wobei diese Formulierung:
o in Umgebungsatmosphäre flüssig ist,
o und bei der Temperatur und/oder den physiologischen pH-Werten und/oder in Gegenwart eines:
* physiologischen Elektrolyts in physiologischer Konzentration,
* und/oder mindestens eines oberflächenaktiven Stoffs
ebenfalls flüssig ist,
o und mindestens einen Wirkstoff PA, bevorzugt in wässriger Lösung, und eine wässrige kolloidale Suspension mit geringer Viskosität umfasst, die auf Submikronpartikeln aus wasserlöslichem biologisch abbaubarem Polymer PO basiert, das hydrophobe Gruppen GH trägt, wobei die Partikeln auf nichtkovalente Art mit dem Wirkstoff PA assoziiert sind, und das Dispersionsmedium der Suspension im Wesentlichen aus Wasser besteht,
**dadurch gekennzeichnet, dass** ihre [PO]-Konzentration auf einen ausreichend hohen Wert festgelegt ist, um die Bildung einer gelatinierten Ablagerung in vitro in Gegenwart mindestens eines Proteins zu ermöglichen.

3. Formulierung nach einem der vorhergehenden Ansprüche, **dadurch gekennzeichnet, dass** ihre [PO]-Konzentration so ist, dass:
■ [PO] ≥ 0,9·C1,
■ bevorzugt 20·C1 ≥ [PO] ≥ C1,
■ und noch besser 10·C1 ≥ [PO] ≥ C1,
wobei C1 für die Konzentration der "induzierten Gelatinierung" der PO-Partikel steht, wie in einem GI-Test gemessen.

4. Formulierung nach einem der vorhergehenden Ansprüche, **dadurch gekennzeichnet, dass** ihre Viskosität kleiner oder gleich 5 Pa.s ist.

5. Formulierung nach einem der vorhergehenden Ansprüche, **dadurch gekennzeichnet, dass** die hydrophoben modifizierten Polymere PO ausgewählt sind aus der Gruppe, umfassend: Polyaminosäuren, Polysaccharide, bevorzugt aus der Untergruppe, die Pullulane und/oder Chitosane und/oder Mucopolysaccharide, Gelatine oder deren Gemische umfasst.

6. Formulierung nach einem der vorhergehenden Ansprüche, **dadurch gekennzeichnet, dass** die hydrophoben Gruppen (GH) seitlich an der Kette liegen.

7. Formulierung nach einem der vorhergehenden Ansprüche, **dadurch gekennzeichnet, dass** das Polymer (PO) eine Polyaminosäure ist, die aus den Asparaginsäureeinheiten und/oder den Glutaminsäureeinheiten gebildet ist, wobei mindestens ein Teil dieser Einheiten Pfropfen trägt, die mindestens eine hydrophobe Gruppe (GH) umfassen.

8. Formulierung nach Anspruch 7, **dadurch gekennzeichnet, dass** das (oder die) PO durch die folgende allgemeine Formel (I) definiert ist (oder sind): 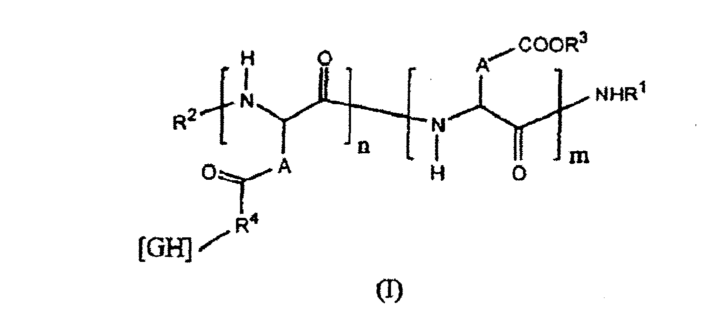 wobei:
■ R¹ für einen H, ein lineares C2-C10-Alkyl oder ein verzweigtes C3-C10-Alkyl, ein Benzyl, eine terminale Aminosäureeinheit oder -R⁴-[GH] steht;
■ R² für einen H, eine lineare C2-C10-Acylgruppe oder verzweigte C3-C10-Acylgruppe, ein Pyroglutamat oder -R⁴-[GH] steht;
■ R³ ein H oder eine kationische Einheit ist, bevorzugt ausgewählt aus der Gruppe, umfassend:
- metallische Kationen, vorteilhafterweise ausgewählt aus der Untergruppe, umfassend: Natrium, Kalium, Calcium, Magnesium,
- organische Kationen, vorteilhafterweise ausgewählt aus der Untergruppe, umfassend:
• Kationen auf der Basis von Amin,
• Kationen auf der Basis von Oligoamin,
• Kationen auf der Basis von Polyamin (wobei Polyethylenimin besonders bevorzugt ist),
• Kationen auf der Basis von Aminosäure(n), vorteilhafterweise ausgewählt aus der Klasse, umfassend Kationen auf der Basis von Lysin oder Arginin,
- oder kationische Polyaminosäuren, vorteilhafterweise ausgewählt aus der Untergruppe, umfassend Polylysin oder Oligolysin;
■ R⁴ für eine direkte Bindung oder einen "Spacer" auf der Basis von 1 bis 4 Aminosäureeinheiten steht;
■ A unabhängig für einen -CH₂-Rest (Asparaginsäureeinheit) oder einen -CH₂-CH₂-Rest (Glutaminsäureeinheit) steht;
■ n/(n+m) als molare Pfropfrate definiert ist und ihr Wert ausreichend niedrig ist, damit PO, das bei 25 °C in Lösung in Wasser mit einem pH-Wert von 7 gegeben wird, eine kolloidale Suspension aus PO-Submikronpartikeln bildet, wobei n/(n + m) bevorzugt im Bereich zwischen 1 und 25 Mol-% und noch besser zwischen 1 und 15 Mol-% liegt;
■ n + m von 10 bis 1.000, bevorzugt zwischen 50 und 300 variiert;
■ GH für eine hydrophobe Gruppe steht.

9. Formulierung nach Anspruch 7, **dadurch gekennzeichnet, dass** das (oder die) PO einer der folgenden allgemeinen Formeln (II), (III) und (IV) entspricht (oder entsprechen): 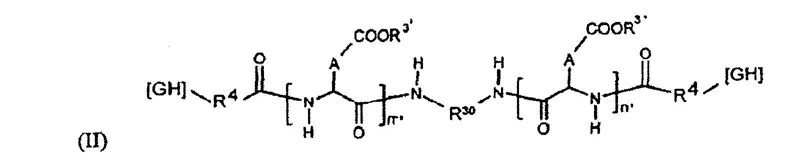 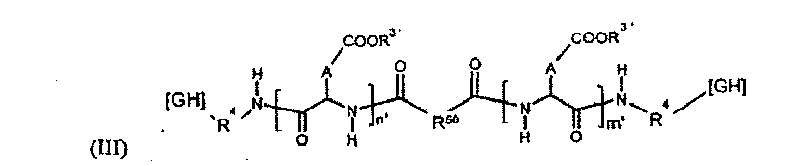 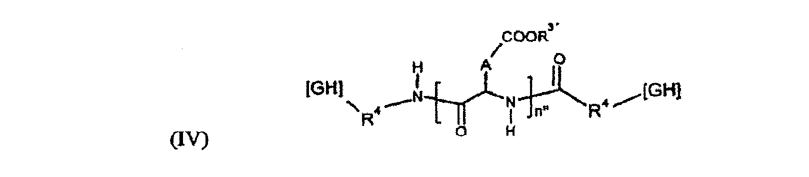 wobei:
■ GH für eine hydrophobe Gruppe steht;
■ R³⁰ eine lineare C2-C6-Alkylgruppe ist;
■ R^{3'} ein H oder eine kationische Einheit ist, bevorzugt ausgewählt aus der Gruppe, umfassend:
- metallische Kationen, vorteilhafterweise ausgewählt aus der Untergruppe, umfassend: Natrium, Kalium, Calcium, Magnesium,
- organische Kationen, vorteilhafterweise ausgewählt aus der Untergruppe, umfassend:
• Kationen auf der Basis von Amin,
• Kationen auf der Basis von Oligoamin,
• Kationen auf der Basis von Polyamin (wobei Polyethylenimin besonders bevorzugt ist),
• Kationen auf der Basis von Aminosäure(n), vorteilhafterweise ausgewählt aus der Klasse, umfassend Kationen auf der Basis von Lysin oder Arginin,
- oder kationische Polyaminosäuren, vorteilhafterweise ausgewählt aus der Untergruppe, umfassend Polylysin oder Oligolysin,
■ R⁵⁰ eine C2-C6-Alkyl-, -Dialcoxy- oder -Diamingruppe ist;
■ R⁴ für eine direkte Bindung oder einen "Spacer" auf der Basis von 1 bis 4 Aminosäureeinheiten steht;
■ A unabhängig für einen -CH₂-Rest (Asparaginsäureeinheit) oder einen -CH₂-CH₂-Rest (Glutaminsäureeinheit) steht;
■ n' + m' oder n" als der Polymerisationsgrad definiert ist und von 10 bis 1.000, bevorzugt zwischen 50 und 300, variiert.

10. Formulierung nach Anspruch 8 oder 9, **dadurch gekennzeichnet, dass** die GH-Gruppen von PO jeweils unabhängig voneinander für einen einwertigen Rest der folgenden Formel stehen: 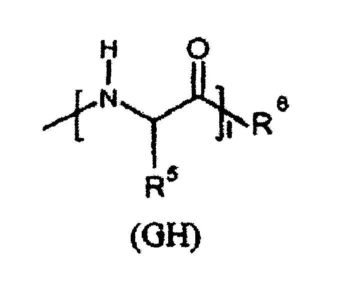 wobei:
- R⁵ für ein Methyl(alanin), Isopropyl(valin), Isobutyl(leucin), Secbutyl(isoleucin), Benzyl(phenylalanin) steht;
- R⁶ für einen hydrophoben Rest steht, der 6 bis 30 Kohlenstoffatome umfasst;
- 1 von 0 bis 6 variiert.

11. Formulierung nach Anspruch 10, **dadurch gekennzeichnet, dass** alle oder ein Teil der hydrophoben Reste R⁶ der PO auf unabhängige Weise ausgewählt sind aus der Gruppe der Reste, umfassend:
■ einen linearen oder verzweigten Alcoxyrest, der 6 bis 30 Kohlenstoffatome umfasst und mindestens ein Heteroatom (bevorzugt O und/oder N und/oder S) und/oder mindestens eine Ungesättigtheit umfassen kann,
■ einen Alcoxyrest, der 6 bis 30 Kohlenstoffatome umfasst und einen oder mehrere annelierte Kohlenstoffringe aufweist und gegebenenfalls mindestens eine Ungesättigtheit und/oder mindestens ein Heteroatom (bevorzugt O und/oder N und/oder S) enthält,
■ einen Alcoxyaryl- oder einen Aryloxyalkylrest mit 7 bis 30 Kohlenstoffatomen und der mindestens eine Ungesättigtheit und/oder mindestens ein Heteroatom (bevorzugt O und/oder N und/oder S) umfassen kann.

12. Formulierung nach Anspruch 10 oder 11, **dadurch gekennzeichnet, dass** der hydrophobe Rest R⁶ des PO-Pfropfs aus einem Alkoholvorprodukt stammt, der ausgewählt ist aus der Gruppe, umfassend: Octanol, Dodecanol, Tetradecanol, Hexadecanol, Octadecanol, Oleylalkohol, Tocopherol oder Cholesterol.

13. Formulierung nach Anspruch 7, **dadurch gekennzeichnet, dass** das PO aus einem alpha-L-Glutamat- oder alpha-L-Glutaminsäurehomopolymer besteht.

14. Formulierung nach Anspruch 7, **dadurch gekennzeichnet, dass** das PO aus einem alpha-L-Aspartat- oder alpha-L-Asparaginsäurehomopolymer besteht.

15. Formulierung nach Anspruch 7, **dadurch gekennzeichnet, dass** das PO aus einem alpha-L-Aspartat-/alpha-L-Glutamat- oder alpha-L-Asparaginsäure/alpha-L-Glutaminsäure-Copolymer besteht.

16. Formulierung nach Anspruch 15, **dadurch gekennzeichnet, dass** in dem PO, die Verteilung der Asparaginsäure- und/oder Glutaminsäureeinheiten, die Träger der Pfropfe tragen, die mindestens ein GH-Muster umfassen, so ist, dass das auf diese Weise gebildete Polymer entweder ein zufälliges Polymer, oder ein Blockpolymer oder ein Multiblockpolymer ist.

17. Formulierung nach Anspruch 1, **dadurch gekennzeichnet, dass** die Molmasse des PO zwischen 2.000 und 100.000 g/Mol, und bevorzugt zwischen 5.000 und 40.000 g/Mol, liegt.

18. Formulierung nach Anspruch 7, **dadurch gekennzeichnet, dass** das PO mindestens eine Pfropf vom Typ Polyalkylenglykol trägt, der an eine Glutamat- und/oder Aspartateinheit gebunden ist.

19. Formulierung nach Anspruch 18, **dadurch gekennzeichnet, dass** der Pfropf vom Typ Polyalkylenglykol die folgende Formel (V) hat: 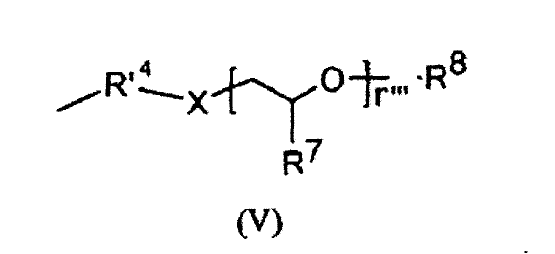 wobei:
- R^{'4} für eine direkte Bindung oder einen "Spacer" auf der Basis von 1 bis 4 Aminosäureeinheiten steht;
- X ein Heteroatom ist, ausgewählt aus der Gruppe, die Sauerstoff, Stickstoff oder einen Schwefel umfasst;
- R⁷ und R⁸ unabhängig für ein H, ein lineares C1-C4-Alkyl stehen;
- n"' von 10 bis 1.000, bevorzugt von 50 bis 300, variiert.

20. Formulierung nach Anspruch 18 oder 19, **dadurch gekennzeichnet, dass** das Polyalkylenglykol ein Polyethylenglykol ist.

21. Formulierung nach einem der Ansprüche 18 bis 20, **dadurch gekennzeichnet, dass** der Molprozentsatz der Polyalkylenglykolpfropfung von 1 bis 30 % variiert.

22. Formulierung nach einem der vorhergehenden Ansprüche, **dadurch gekennzeichnet, dass** der PA ein Protein, ein Glykoprotein, ein Protein, das an eine oder mehrere Polyalkylenglykolketten gebunden ist [bevorzugt Polyethylenglykol (PEG): "PEGyliertes Protein"], ein Polysaccharid, ein Liposaccharid, ein Oligonucleotid, ein Polynucleotid oder ein Peptid ist,
bevorzugt ausgewählt aus Hämoglobinen, Cytochromen, Albuminen, Interferonen, Cytokinen, Antigenen, Antikörpern, Erythropoetin, Insulin, Wachstumshormonen, den Faktoren VIII und IX, Interleukinen oder deren Gemischen, den die Hämatopoese stimulierenden Faktoren.

23. Formulierung nach einem der Ansprüche 1 bis 21, **dadurch gekennzeichnet, dass** der Wirkstoff ein "kleines" hydrophobes, hydrophiles oder amphiphiles organisches Molekül ist.

24. Formulierung nach einem der Ansprüche 1 bis 22, **dadurch gekennzeichnet, dass** ihr Masseanteil an nicht mit Submikronpartikeln assoziiertem PA [nicht assoziierter PA] in Gew.-% so ist, dass:
o [nicht assoziierter PA] ≤ 1
o bevorzugt [nicht assoziierter PA] ≤ 0,5.

25. Formulierung nach einem der vorhergehenden Ansprüche, **dadurch gekennzeichnet, dass** sie auf parenteralem, subkutanem, intramuskulärem, intradermalem, intraperitonealem, intrazerebralem Weg oder in einen Tumor injizierbar ist.

26. Formulierung nach einem der vorhergehenden Ansprüche, **dadurch gekennzeichnet, dass** sie zur Herstellung von Medikamenten dient, insbesondere zur parenteralen, subkutanen, intramuskulären, intradermalen, intraperitonealen, intrazerebralen Verabreichung oder zur Verabreichung in einen Tumor sogar auf oralem, nasalem, vaginalem oder okularem Weg.

27. Verfahren zur Herstellung von Medikamenten, insbesondere zur parenteralen, subkutanen, intramuskulären, intradermalen, intraperitonealen, intrazerebralen Verabreichung oder der Verabreichung in einen Tumor sogar auf oralem, nasalem, vaginalem oder okularem Weg, **dadurch gekennzeichnet, dass** es im Wesentlichen darin besteht, mindestens eine Formulierung nach einem der Ansprüche 1 bis 26 einzusetzen.

28. Abgeleitetes Produkt, **dadurch gekennzeichnet, dass** es Submikronpartikel umfasst, die durch die nichtkovalenten PO/PA-Assoziationen, wie in Anspruch 1 definiert, gebildet werden und **dadurch**, dass es aus der Formulierung nach einem der Ansprüche 1 bis 26 erhalten wird.

29. Abgeleitetes Produkt nach Anspruch 28, **dadurch gekennzeichnet, dass** es aus einem Pulver oder aus einem Gel besteht.

30. Verfahren zur Herstellung der Formulierung nach einem der Ansprüche 1 bis 26, **dadurch gekennzeichnet, dass** es im Wesentlichen darin besteht:
◆ eine kolloidale Suspension aus Nanopartikeln mindestens eines PO einzusetzen,
◆ diese kolloidale Suspension aus PO-Nanopartikeln mit mindestens einem PA, bevorzugt in wässriger Lösung, zu mischen,
◆ gegebenenfalls mindestens einen Exzipienten zuzufügen,
◆ nötigenfalls den pH-Wert und/oder die Osmolarität anzupassen, und
◆ gegebenenfalls die so erhaltene Suspension zu filtrieren.

31. Verfahren nach Anspruch 30, **dadurch gekennzeichnet, dass** der (oder die) PA(s) in Form einer Suspension oder einer wässrigen Lösung zum Mischen mit der kolloidalen Suspension aus PO-Nanopartikeln vorliegt (oder vorliegen).

32. Verfahren zur Herstellung der Formulierung nach einem der Ansprüche 1 bis 26, **dadurch gekennzeichnet, dass** es im Wesentlichen darin besteht:
ein Pulver aus Nanopartikeln mindestens eines PO einzusetzen,
dieses Pulver mit einer Suspension oder wässrigen Lösung aus mindestens einem PA, bevorzugt in wässriger Lösung, zu mischen,
gegebenenfalls mindestens einen Exzipienten zuzufügen,
nötigenfalls den pH-Wert und/oder die Osmolarität anzupassen, und
gegebenenfalls die so erhaltene Suspension zu filtrieren.

33. Verfahren zur Herstellung der Formulierung nach einem der Ansprüche 1 bis 26, **dadurch gekennzeichnet, dass** es im Wesentlichen darin besteht:
ein Pulver einzusetzen, das aus dem Trocknen der flüssigen Formulierung nach einem der Ansprüche 1 bis 26 stammt,
dieses Pulver mit einem wässrigen flüssigen Medium, bevorzugt unter Rühren, zu mischen,
gegebenenfalls mindestens einen Exzipienten zuzufügen,
nötigenfalls den pH-Wert und/oder die Osmolarität anzupassen, und
gegebenenfalls die so erhaltene Suspension zu filtrieren.

34. Verfahren zur Herstellung eines Pulvers, das von der Formulierung nach einem der Ansprüche 1 bis 26 abgeleitet ist, **dadurch gekennzeichnet, dass** das Pulver durch Trocknen der Formulierung nach einem der Ansprüche 1 bis 26 erhalten wird.
